# EUROPEAN PATENT APPLICATION

(11) **EP 1 505 064 A1**
(43) Date of publication of application: **09.02.2005**
(21) Application number: 03017810.7
(22) Date of filing: 05.08.2003
(51) Int. Cl.: C07D 239/04, A61K 31/505, A61P 19/00, A61P 29/00

(54) **2-Aminopyrimidine derivatives**

(71) Applicant: Bayer HealthCare AG, 51368 Leverkusen (DE)
(72) Inventor: Sato, Hiroki, Nara-shi Nara-ken, 531-0804 (JP); Fukushima, Keiko, Nara-shi Nara-ken, 631-0801 (JP); Shimazaki, Makoto, Kobe-shi Hyogo-ken, 657-0024 (JP); Urbahns, Klaus, Kobe-shi Hyogo-ken, 657-0024 (JP); Sakai, Katsuya, Daito-city Osaka, 574-0003 (JP); Gantner, Florian, Osaka-shi Osaka-fu, 543-0033 (JP); Bacon, Kevin, Kobe-shi Hyogo-ken, 658-0032 (JP)

(57) **Abstract**

The present invention relates to novel 2-aminopyrimidine derivatives, processes for preparing them and pharmaceutical preparations containing them. The 2-aminopyrimidine derivatives of the present invention exhibit enhanced potency for histamine H4 receptor antagonism and can be used for the prophylaxis and treatment of diseases associated with histamine H4 receptor activity.

More specifically, the 2-aminopyrimidine derivatives of the present invention are useful for treatment and prophylaxis of diseases as follows: asthma, rhinitis, allergic diseases, chronic obstructed pulmonary disease (CORD), atherosclerosis and rheumatoid arthritis. wherein 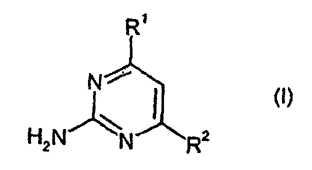 R¹ is 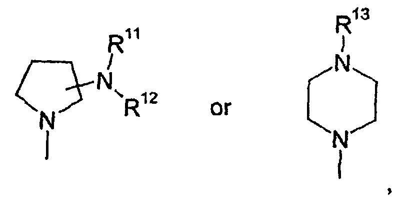 R² is phenyl or naphthyl.

## Description

### DETAILED DESCRIPTION OF INVENTION

### Technical Field

The present invention relates to novel 2-aminopyrimidine derivatives, processes for preparing them and pharmaceutical preparations containing them. The 2-aminopyrimidine derivatives of the present invention exhibit enhanced potency for histamine H4 receptor antagonism and can be used for the prophylaxis and treatment of diseases associated with histamine H4 receptor activity.

More specifically, the 2-aminopyrimidine derivatives of the present invention are useful for treatment and prophylaxis of diseases as follows: asthma, rhinitis, allergic diseases, chronic obstructed pulmonary disease (CORD), atherosclerosis and rheumatoid arthritis.

### Background Art

Histamine exerts its numerous physiological and pathophysiological functions by acting on its receptor entities which belong to the heptahelical G-protein coupled receptor family. (Hough LB, Mol Pharmacol, 59:415-419, 2001.) The specific functions of H1, H2 and H3 subtypes are well characterized (reviewed in the following references: Bakker RA et al., Clin Allergy Immunol, 17:27-64, 2002; Repka-Ramirez MS and Baraniuk JN, Clin Allergy Immunol, 17:1-25, 2002; Winbery SL and Lieberman PL, Clin Allergy Immunol, 17:287-317, 2002; Barocelli E and Ballabeni V, Pharmacol Res, 47:299-304, 2003.) and antagonists to each subtype are identified and some drugs have been clinically used. The fourth member, H₄ has recently been cloned and characterized by various groups (Oda T et al., J. Biol. Chem. 275(47):36781-36786, 2000; Nakamura T et al, Biochem Biophys Res Commun 279:615-620, 2000; Zhu et al., Mol. Pharmacol. 59(3):434-441, 2001; Liu C et al., Mol. Pharmacol. 59:420-426, 2001; Morse et al., J. Pharmacol. Exp. Ther. 296(3):1058-1066, 2001; Nguyen et al., Mol. Pharmacol. 59:427-433, 2001; O'Reilly M et al, J Recept Signal Transduct Res 22:431-448, 2002.) The predominant expression of H4 on leukocyte populations suggests potential immunological and inflammatory functions. Indeed, some inflammatory functions mediated by H4 have been recently identified *in vitro.* Chemotaxis of human eosinophils (O'Reily et al, above), IL-16 release from human CD8⁺ lymphocytes (Gantner F et al, J Pharmacol Exp Ther 303:300-307, 2002.), and calcium mobilization and chemotaxis of murine mast cells (Hofstra CL, et al J Pharmacol Exp Ther. 305(3):1212-21, 2003) are controlled by H4. Also, Considerable expression of H4 is also seen on lung tissue and lung cells (Coge F, et al., Biochem Biophys Res Commun 284:301-309, 2001; Morse et al., see above; Zhu et al., see above; Gantner F et al, see above.) which makes H4 an attractive target for inflammatory lung disorders.

It is therefore expected that antagonists of histamine H4 receptor can be used for prophylaxis and treatment of immunological or inflammatory disorders/diseases, including asthma, rhinitis, allergic diseases, chronic obstructed pulmonary disease (CORD), atherosclerosis and rheumatoid arthritis.

WO01/047921 discloses pyrimidine derivatives represented by the general formula: 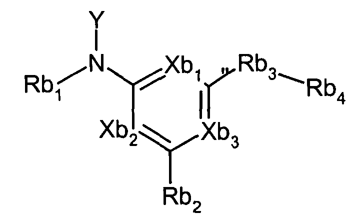 wherein
- Rb₁: is hydrogen, alkyl, alkoxyalkyl, arylalkyl, heteroaryl, heteroarylalkyl etc;
- Rb₂: is halogen, alkyl, OH, (un)substituted heteroaryl, (un)substituted heterocyclyl, etc;
- Rb₃: is bond, 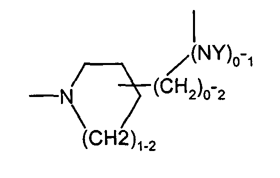 etc;
- Rb₄: is hydrogen, halogen, alkyl, heterocyclyl, alkylamino, etc;
- Xb₁, Xb₂ and Xb₃: independently represent -N= or -C(H)- ;
- Y: is hydrogen, alkyl, or Y and Rb₁ together with N to form (un)substituted heterocyclyl or heteroaryl;
- X, X1 and X2: are -N= or -C(H)- ;
as a protein kinase inhibitor.

WO90/12790 discloses pyrimidine derivatives represented by the general formula: 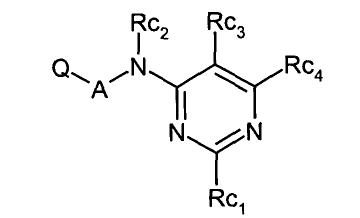 wherein
- Rc₁ and Rc₄: independently represent hydrogen, amino, (un)substituted phenyl etc;
- Rc₂: is hydrogen, alkyl, alkenyl, etc ,or alkylene or alkenylene to form ring together with Q-A-N, etc;
- Rc₃: is H, alkyl or alkenyl;
- A: is direct bond, alkylene or oxy-alkylene;
- Q: is pyridyl, furyl, thienyl or (un)substituted phenyl.
as an intermediate for a cardiovascular agent.

WO01/062233 discloses pyrimidine derivatives represented by the general formula: 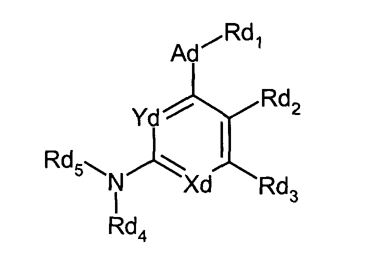
- Ad: is bond, -N(R)-, etc;
- Rd₁: is hydrogen, -(CH₂)ₙ-phenyl, -(CH₂)ₙ-piperazin-4-yl optionally substituted by lower alkyl, phenyl, -(CH₂)ₙ-piperidinyl optionally substituted by lower alkyl, -(CH₂)ₙ-NH-phenyl etc;
- n: is an integer of 0-4;
- Rd₂: is hydrogen, halogen, cyano, alkyl, etc;
- Rd₃: is lower alkyl, phenyl optionally substituted by alkyl, alkoxy or halogen, heterocycles (eg. thienyl, furyl) etc;
- Rd₄ and Rd₅: independently represent hydrogen, optionally substituted phenyl, etc.
as an adenosine receptor modulators.

EP459830 discloses pyrimidine derivatives represented by the general formula: 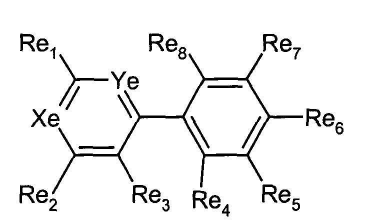 wherein
- Re₁ and Re₂: are independently OH or N Re₉ Re₁₀;
- Re₉ and Re₁₀: independently represent hydrogen, alkyl etc, or together with nitrogen to form a ring, which is optionally substituted by alkyl, aryl or arylalkyl;
- Re₃: is hydrogen, haloalkyl, halogen, alkyl, etc;
- Re₄, Re₅, Re₆, Re₇ and Re₈: independently represent hydrogen, halogen, amino sulphonamido, or alkylsulphonylamino, with the proviso that at least one of R e₄-R e₈ is not hydrogen.
as a glutamate release inhibitor.

Also, WO02/072548 discloses indole derivatives as histamine H4 receptor antagonist.

However, none of the references and other reference disclose 2-aminopyrimidine derivatives having histamine H4 receptor antagonistic activity.

The development of a compound which has effective anti-inflammatory actions based on a specific and selective antagonistic activity to histamine H4 receptor and can be used for the prophylaxis and treatment of diseases associated with histamine H4 receptor activity has been still desired.

### Summary of the invention

As a result of extensive studies on chemical modification of 2-aminopyrimidine derivatives, the present inventors have found that the compounds of novel chemical structure related to the present invention have unexpectedly excellent antagonistic activity to histamine H4 receptor. The present invention has been accomplished based on these findings.

This invention is to provide novel 2-aminopyrimidine derivatives of the formula (I) their tautomeric and stereoisomeric forms, and salts thereof. 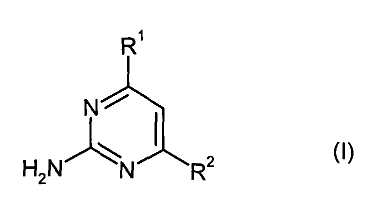 wherein
- R¹: is 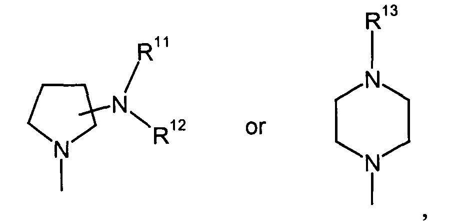 wherein
R¹¹ and R¹² independently represent hydrogen or C₁₋₆alkyl optionally substituted by halogen, cyano, hydroxy, carboxy, amino, C₁₋₆alkylamino, N,N-di(C₁₋₆alkyl)amino, C₁₋₆alkylthio, C₁₋₆₋ alkoxy, or C₃₋₈cycloalkyl;
R¹³ is C₁₋₆alkyl optionally substituted by halogen, cyano, hydroxy, carboxy, amino, C₁₋₆alkylamino, N,N-di(C₁₋₆alkyl)amino, C₁₋₆₋ alkylthio, C₁₋₆alkoxy, or C₃₋₈cycloalkyl;
- R²: is phenyl or naphthyl,
wherein
said phenyl and naphthyl are optionally having one or more substituents selected from the group consisting of halogen, amino, carboxy, cyano, hydroxy, nitro, phenyl, C₁₋₆alkanoyl, N-(C₁₋₆alkyl)sulfonylamino, N-phenylsulfonylamino, C₁₋₆alkylamino, N,N-di(C₁₋₆alkyl)-amino, N-(C₁₋₆alkanoyl)amino, C₁₋₆alkoxycarbonyl, C₁₋₆alkylthio, N-(C₁₋₆alkoxycabonyl)amino, N-arylamino, N-(aryl C₁₋₆alkyl)amino, aminocarbonyl, N-(C₁₋₆alkyl)aminocarbonyl, N,N-di(C₁₋₆alkyl)aminocarbonyl, C₃₋₈cycloalkyl, C₁₋₆alkylsulfonyl, sulfamoyl, aryl C₁₋₆alkoxycarbonyl, C₁₋₆alkyl, C₁₋₆alkyl substituted by cyano, hydroxy, carboxy, amino, C₁₋₆alkylamino, N,N-di(C₁₋₆alkyl)amino, C₁₋₆alkylthio, C₁₋₆alkoxy, or mono-, di-, or tri- halogen, C₁₋₆alkoxy, C₁₋₆alkoxy substituted by mono-, di-, or tri- halogen, -N(R²¹)C(O)N(R²²)(R²³), and -N(R²¹)C(O) R²⁴,
with the proviso that when R¹ is 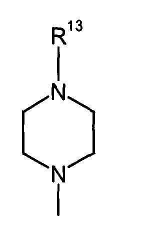 and R² is optionally substituted phenyl, said phenyl ring is substituted by at least one substituent selected from the group consisting of carboxy, cyano, hydroxy, phenyl, C₁₋₆alkanoyl, N-phenylsulfonylamino, C₁₋₆alkylamino, N,N-di(C₁₋₆alkyl)amino, N-(C₁₋₆alkanoyl)-amino, C₁₋₆alkoxycarbonyl, C₁₋₆alkylthio, N-(C₁₋₆alkoxycabonyl)-amino, N-arylamino, N-(aryl C₁₋₆alkyl)amino, aminocarbonyl, N-(C₁₋₆alkyl)aminocarbonyl, N,N-di(C₁₋₆alkyl)aminocarbonyl, C₃₋₈cycloalkyl, C₁₋₆alkylsulfonyl, aryl C₁₋₆alkoxycarbonyl, C₁₋₆alkyl substituted by cyano, hydroxy, carboxy, amino, C₁₋₆alkylamino, N,N-di-(C₁₋₆alkyl)amino, C₁₋₆alkylthio, C₁₋₆alkoxy, or mono-, di-, or trihalogen, C₁₋₆alkoxy substituted by mono-, di-, or tri- halogen, -N(R²¹)C(O)N(R²²)(R²³), and -N(R²¹)C(O) R²⁴,
wherein
R²¹ is hydrogen or C₁₋₆alkyl;
R²² is C₁₋₆alkyl, or phenyl optionally having one or more substituents selected from the group consisting of halogen, amino, carbamoyl, cyano, hydroxy, nitro, phenyl, C₁₋₆alkanoyl, C₁₋₆alkyl optionally substituted by hydroxy or mono-, di-, or tri- halogen, and C₁₋₆-alkoxy optionally substituted by mono-, di-, or trihalogen;
R²³ is hydrogen or C₁₋₆alkyl;
R²⁴ is phenyl optionally having one or more substituents selected from the group consisting of halogen, amino, carboxy, carbamoyl, cyano, hydroxy, nitro, phenyl, C₁₋₆alkanoyl, C₁₋₆alkyl optionally substituted by hydroxy or mono-, di-, or tri- halogen, and C₁₋₆alkoxy optionally substituted by mono-, di-, or tri- halogen.

In another embodiment, the present invention provides 2-aminopyrimidine derivatives of the formula (I-i), its tautomeric or stereoisomeric form, or a salt thereof: 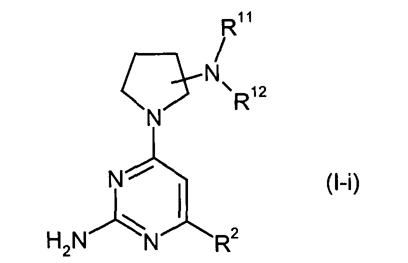 wherein
- R¹¹ and R¹²: independently represent hydrogen or C₁₋₆alkyl optionally substituted by halogen, cyano, hydroxy, carboxy, amino, C₁₋₆alkylamino, N,N-di(C₁₋₆alkyl)amino, C₁₋₆alkylthio, C₁₋₆alkoxy, C₃₋₈cycloalkyl;
- R²: is phenyl or naphthyl,
wherein
said phenyl and naphthyl are optionally having one or more substituents selected from the group consisting of halogen, amino, carboxy, cyano, hydroxy, nitro, phenyl, C₁₋₆alkanoyl, N-(C₁₋₆alkyl)-sulfonylamino, N-phenylsulfonylamino C₁₋₆alkylamino, N,N-di(C₁₋₆alkyl)amino, N-(C₁₋₆alkanoyl)amino, C₁₋₆alkoxycarbonyl, C₁₋₆-alkylthio, N-(C₁₋₆alkoxycabonyl)amino, N-arylamino, N-(aryl-C₁₋₆alkyl)amino, aminocarbonyl, N-(C₁₋₆alkyl)aminocarbonyl, N,N-di(C₁₋₆alkyl)aminocarbonyl, C₃₋₈cycloalkyl, C₁₋₆alkylsulfonyl, sulfamoyl, aryl C₁₋₆alkoxycarbonyl, C₁₋₆alkyl substituted by cyano, hydroxy, carboxy, amino, C₁₋₆alkylamino, N,N-di(C₁₋₆alkyl)amino, C₁₋₆alkylthio, C₁₋₆alkoxy, or mono-, di-, or tri- halogen, C₁₋₆alkoxy optionally substituted by mono-, di-, or tri- halogen, N(R²¹)C(O)N(R²²)(R²³), and N(R²¹)C(O) R²⁴,
wherein
R²¹ is hydrogen or C₁₋₆alkyl;
R²² is C₁₋₆alkyl, or phenyl optionally having one or more substituents selected from the group consisting of halogen, amino, carbamoyl, cyano, hydroxy, nitro, phenyl, C₁₋₆alkanoyl, C₁₋₆alkyl optionally substituted by hydroxy or mono-, di-, or tri- halogen, and C₁₋₆alkoxy optionally substituted by mono- di-, or trihalogen;
R²³ is hydrogen or C₁₋₆alkyl; and
R²⁴ is phenyl optionally having one or more substituents selected from the group consisting of halogen, amino, carboxy, carbamoyl, cyano, hydroxy, nitro, phenyl, C₁₋₆alkanoyl, C₁₋₆alkyl optionally substituted by hydroxy or mono-, di-, or tri- halogen, and C₁₋₆alkoxy optionally substituted by mono-, di-, or tri- halogen.

In another embodiment, the present invention provides 2-aminopyrimidine derivatives of the formula (I-i), its tautomeric or stereoisomeric form, or a salt thereof:
wherein
- R²: is phenyl or naphthyl,
wherein
said phenyl and naphthyl are optionally having one or more substituents selected from the group consisting of halogen, amino, cyano, hydroxy, nitro, phenyl, C₁₋₆alkanoyl, C₁₋₆alkylamino, N,N-di(C₁₋₆alkyl)amino, N-(C₁₋₆alkanoyl)amino, C₁₋₆alkoxycarbonyl, C₁₋₆-alkylthio, C₁₋₆alkylsulfonyl, C₁₋₆alkyl, trifluoromethyl, C₁₋₆alkyl substituted by cyano, hydroxy, amino, C₁₋₆alkylamino, N,N-di-(C₁₋₆alkyl)amino, or C₁₋₆alkoxy, C₁₋₆alkoxy, N(R²¹)C(O)N(R²²)(R²³), and N(R²¹)C(O) R²⁴,
wherein
R²¹ is hydrogen or C₁₋₆alkyl;
R²² is C₁₋₆alkyl, or phenyl optionally having one or more substituents selected from the group consisting of halogen, amino, carbamoyl, cyano, hydroxy, nitro, phenyl, C₁₋₆alkanoyl, C₁₋₆alkyl optionally substituted by hydroxy or mono-, di-, or tri- halogen, and C₁₋₆alkoxy optionally substituted by mono- di-, or trihalogen;
R²³ is hydrogen or C₁₋₆alkyl; and
R²⁴ is phenyl optionally having one or more substituents selected from the group consisting of halogen, amino, carboxy, carbamoyl, cyano, hydroxy, nitro, phenyl, C₁₋₆alkanoyl, C₁₋₆alkyl optionally substituted by hydroxy or mono-, di-, or tri- halogen, and C₁₋₆alkoxy optionally substituted by mono-, di-, or tri- halogen.

In another embodiment, the present invention provides 2-aminopyrimidine derivatives of the formula (I-i), its tautomeric or stereoisomeric form, or a salt thereof:
wherein
- R¹¹ and R¹²: independently represent hydrogen or methyl.

More preferably, said 2-aminopyrimidine derivative of the formula (I-i) is selected from the group consisting of:
4-(3-aminopyrrolidin-1-yl)-6-phenylpyrimidin-2-amine trihydrochloride;
4-[3-(dimethylamino)pyrrolidin-1-yl]-6-phenylpyrimidin-2-amine trihydrochloride;
4-[3-(methylamino)pyrrolidin-1-yl]-6-phenylpyrimidin-2-amine trihydrochloride;
4-[3-(methylamino)pyrrolidin-1-yl]-6-(3-nitrophenyl)pyrimidin-2-amine trihydrochloride;
4-[(3R)-3-(methylamino)pyrrolidin-1-yl]-6-(3-nitrophenyl)pyrimidin-2-amine trihydrochloride;
4-[(3 S)-3-(methylamino)pyrrolidin-1-yl]-6-(3-nitrophenyl)pyrimidin-2-amine trihydrochloride;
4-[(3S)-3-(methylamino)pyrrolidin-1-yl]-6-phenylpyrimidin-2-amine trihydrochloride;
4-[(3R)-3-(methylamino)pyrrolidin-1 -yl]-6-phenylpyrimidin-2-amine trihydrochloride;
4-[3-(methylamino)pyrrolidin-1-yl]-6-(3-methylphenyl)pyrimidin-2-amine trihydrochloride;
1-(3- {2-amino-6-[3-(methylamino)pyrrolidin-1-yl]pyrimidin-4-yl}phenyl)-ethanone trihydrochloride;
3-{2-amino-6-[3-(methylamino)pyrrolidin-1-yl]pyrimidin-4-yl}phenol trihydrochloride;
(3-{2-amino-6-[3-(methylamino)pyrrolidin-1-yl]pyrimidin-4-yl}phenyl)-methanol trihydrochloride; and
3- {2-amino-6-[3-(methylamino)pyrrolidin-1-yl]pyrimidin-4-yl}benzonitrile trihydrochloride.

In another embodiment, the present invention provides 2-aminopyrimidine derivatives of the formula (I-ii), its tautomeric or stereoisomeric form, or a salt thereof: 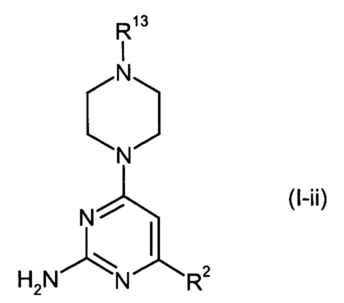 wherein
- R¹³: is C₁₋₆alkyl optionally substituted by halogen, cyano, hydroxy, carboxy, amino, C₁₋₆alkylamino, N,N-di(C₁₋₆alkyl)amino, C₁₋₆alkylthio, C₁₋₆alkoxy, C₃₋₈cycloalkyl;
- R²: is phenyl having one or more substituents selected from the group consisting of carboxy, cyano, hydroxy, phenyl, C₁₋₆alkanoyl, N-(C₁₋₆alkyl)sulfonylamino, N-phenylsulfonylamino, C₁₋₆alkylamino, N,N-di(C₁₋₆alkyl)amino, N-(C₁₋₆alkanoyl)amino, C₁₋₆alkoxycarbonyl, C₁₋₆alkylthio, N-(C₁₋₆alkoxycabonyl)amino, N-arylamino, N-(aryl C₁₋₆alkyl)amino, aminocarbonyl, N-(C₁₋₆alkyl)aminocarbonyl, N,N-di(C₁₋₆alkyl)aminocarbonyl, C₃₋₈cycloalkyl, C₁₋₆alkylsulfonyl, aryl C₁₋₆alkoxycarbonyl, C₁₋₆alkyl substituted by cyano, hydroxy, carboxy, amino, C₁₋₆alkylamino, N,N-di(C₁₋₆alkyl)amino, C₁₋₆alkylthio, C₁₋₆-alkoxy, or mono-, di-, or tri- halogen, C₁₋₆alkoxy substituted by mono-, di-, or tri- halogen, -N(R²¹)C(O)N(R²²)(R²³), and N(R²¹)C(O) R²⁴, or naphthyl optionally having one or more substituents selected from the group consisting of halogen, amino, carboxy, cyano, hydroxy, nitro, phenyl, C₁₋₆alkanoyl, N-phenylsulfonylamino, C₁₋₆alkylamino, N,N-di(C₁₋₆alkyl)amino, N-(C₁₋₆alkanoyl)amino, C₁₋₆alkoxycarbonyl, C₁₋₆-alkylthio, N-(C₁₋₆alkoxycabonyl)amino, N-arylamino, N-(aryl C₁₋₆-alkyl)amino, aminocarbonyl, N-(C₁₋₆alkyl)aminocarbonyl, N,N-di(C₁₋₆alkyl)aminocarbonyl, C₃₋₈cycloalkyl, C₁₋₆alkylsulfonyl, sulfamoyl, aryl C₁₋₆alkoxycarbonyl, C₁₋₆alkyl optionally substituted by cyano, hydroxy, carboxy, amino, C₁₋₆alkylamino, N,N-di(C₁₋₆alkyl)-amino, C₁₋₆alkylthio, C₁₋₆alkoxy, or mono-, di-, or tri- halogen, C₁₋₆alkoxy optionally substituted by amono-, di-, or tri- halogen, -N(R²¹)C(O)N(R²²)(R²³), and N(R²¹)C(O) R²⁴,
wherein
- R²¹: is hydrogen or C₁₋₆alkyl;
- R²²: is C₁₋₆alkyl, or phenyl optionally having one or more substituents selected from the group consisting of halogen, amino, carbamoyl, cyano, hydroxy, nitro, phenyl, C₁₋₆alkanoyl, C₁₋₆-alkyl optionally substituted by hydroxy or mono-, di-, or trihalogen, and C₁₋₆alkoxy optionally substituted by mono-, di-, or tri- halogen;
- R²³: is hydrogen or C₁₋₆alkyl;
- R²⁴: is phenyl optionally having one or more substituents selected from the group consisting of halogen, amino, carboxy, carbamoyl, cyano, hydroxy, nitro, phenyl, C₁₋₆alkanoyl, C₁₋₆-alkyl optionally substituted by hydroxy or mono-, di-, or trihalogen, and C₁₋₆alkoxy optionally substituted by amono-, di-, or tri- halogen.

In another embodiment, the present invention provides 2-aminopyrimidine derivatives of the formula (I-ii), its tautomeric or stereoisomeric form, or a salt thereof:
wherein
- R²: is phenyl having one or more substituents selected from the group consisting of cyano, hydroxy, phenyl, C₁₋₆alkanoyl, C₁₋₆alkylamino, N,N-di(C₁₋₆alkyl)amino, N-(C₁₋₆alkanoyl)amino, C₁₋₆alkoxycarbonyl, C₁₋₆alkylthio, C₁₋₆alkylsulfonyl, trifluoromethyl, C₁₋₆alkyl substituted by cyano, hydroxy, amino, C₁₋₆alkylamino, N,N-di(C₁₋₆alkyl)amino, or C₁₋₆alkoxy, and C₁₋₆alkoxy, or naphthyl optionally having one or more substituents selected from the group consisting of halogen, amino, cyano, hydroxy, nitro, phenyl, C₁₋₆alkanoyl, C₁₋₆alkylamino, N,N-di(C₁₋₆alkyl)amino, N-(C₁₋₆alkanoyl)amino, C₁₋₆alkoxycarbonyl, C₁₋₆alkylthio, C₁₋₆alkylsulfonyl, C₁₋₆alkyl, trifluoromethyl, C₁₋₆alkyl substituted by cyano, hydroxy, amino, C₁₋₆alkylamino, N,N-di-(C₁₋₆alkyl)atnino, or C₁₋₆alkoxy, and C₁₋₆alkoxy.

More preferably, said 2-aminopyrimidine derivative of the formula (I-ii) is selected from the group consisting of:
3-[2-amino-6-(4-methylpiperazin-1-yl)pyrimidin-4-yl]phenol;
1-{3-[2-amino-6-(4-methylpiperazin-1-yl)pyrimidin-4-yl]phenyl}ethanone;
{3-[2-amino-6-(4-methylpiperazin-1-yl)pyrimidin-4-yl]phenyl}methanol;
4-(4-methylpiperazin-1-yl)-6-[3-(trifluoromethyl)phenyl]pyrimidin-2-amine;
4-biphenyl-3-yl-6-(4-methylpiperazin-1-yl)pyrimidin-2-amine trihydrochloride;
4-[3-(dimethylamino)phenyl]-6-(4-methylpiperazin-1-yl)pyrimidin-2-amine;
4-(4-methylpiperazin-1-yl)-6-(1-naphthyl)pyrimidin-2-amine; and
3-[2-amino-6-(4-methylpiperazin-1-yl)pyrimidin-4-yl]benzonitrile.

Further this invention is to provide a use of the 2-aminopyrimidine derivatives of the formula (I), its tautomeric or stereoisomeric form, or a physiologically acceptable salt thereof in the preparation of a medicament. Preferably, said medicament is suitable for treating or preventing a disorder or disease associated with histamine H4 receptor activity.

The 2-aminopyrimidine derivatives of the formula (I), its tautomeric or stereoisomeric form, or a physiologically acceptable salt thereof surprisingly show excellent histamine H4 receptor antagonistic activity. They are, therefore, suitable for the production of medicament or medical composition, which may be useful to treat histamine H4 receptor related diseases.

More specifically, compounds of the formula (I) are effective for the treatment or prevention of an inflammatory disorder or disease such as asthma, rhinitis, allergic diseases or chronic obstructed pulmonary disease (CORD).

Also, compounds of the formula (I) are effective for the treatment or prevention of an immunological disorder or disease such as rheumatoid arthritis or atherosclerosis.

Further, the present invention provides a medicament, which includes one of the compounds described above and optionally pharmaceutically acceptable excipients.

The Alkyl per se and "alk" and "alkyl" in alkoxy, alkanoyl, alkylamino, alkylaminocarbonyl, alkylaminosulphonyl, alkylsulphonylamino, alkoxycarbonyl, alkoxycarbonylamino and alkanoylamino represent a linear or branched alkyl radical having generally 1 to 6, preferably 1 to 4 and particularly preferably 1 to 3 carbon atoms, representing illustratively and preferably methyl, ethyl, n-propyl, isopropyl, tert-butyl, n-pentyl and n-hexyl.

Alkoxy illustratively and preferably represents methoxy, ethoxy, n-propoxy, iso-propoxy, tert-butoxy, n-pentoxy and n-hexoxy.

Alkanoyl illustratively and preferably represents acetyl and propanoyl.

Alkylamino represents an alkylamino radical having one or two (independently selected) alkyl substituents, illustratively and preferably representing methylamino, ethylamino, n-propylamino, isopropylamino, tert-butylamino, n-pentylamino, n-hexyl-amino, N,N-dimethylamino, N,N-diethylamino, N-ethyl-N-methylamino, N-methyl-N-n-propylamino, N-isopropyl-N-n-propylamino, N-t-butyl-N-methylamino, N-ethyl-N-n-pentylamino and N-n-hexyl-N-methylamino.

Alkylaminocarbonyl or alkylcarbamoyl represents an alkylaminocarbonyl radical having one or two (independently selected) alkyl substituents, illustratively and preferably representing methylaminocarbonyl, ethylaminocarbonyl, n-propylaminocarbonyl, isopropylamino-carbonyl, tert-butylaminocarbonyl, n-pentylaminocarbonyl, n-hexylaminocarbonyl, N,N-dimethylaminocarbonyl, N,N-diethylaminocarbonyl, N-ethyl-N-methylaminocarbonyl, N-methyl-N-n-propylaminocarbonyl, N-isopropyl-N-n-propylaminocarbonyl, N-t-butyl-N-methylaminocarbonyl, N-ethyl-N-n-pentylamino-carbonyl and N-n-hexyl-N-methylaminocarbonyl.

Alkylaminosulphonyl represents an alkylaminosulphonyl radical having one or two (independently selected) alkyl substituents, illustratively and preferably representing methylaminosulphonyl, ethylaminosulphonyl, n-propylaminosulphonyl, isopropylaminosulphonyl, tert-butylaminosulphonyl, n-pentylaminosulphonyl, n-hexyl-aminosulphonyl, N,N-dimethylaminosulphonyl, N,N-diethylaminosulphonyl, N-ethyl-N-methylamino-sulphonyl, N-methyl-N-n-propylaminosulphonyl, N-isopropyl-N-n-propylaminosulphonyl, N-t-butyl-N-methylaminosulphonyl, N-ethyl-N-n-pentylaminosulphonyl and N-n-hexyl-N-methylaminosulphonyl.

Alkylsulphonylamino illustratively and preferably represents methylsulphonylamino, ethylsulphonylamino, n-propylsulphonylamino, isopropylsulphonylamino, tert-butylsulphonylamino, n-pentylsulphonylamino and n-hexylsulphonylamino.

Alkoxycarbonyl illustratively and preferably represents methoxycarbonyl, ethoxycarbonyl, n-propoxycarbonyl, isopropoxycarbonyl, tert-butoxycarbonyl, n-pentoxycarbonyl and n-hexoxycarbonyl. Alkoxycarbonylamino illustratively and preferably represents methoxycarbonylamino, ethoxycarbonylamino, n-propoxycarbonylamino, isopropoxycarbonylamino, tert-butoxycarbonylamino, n-pentoxycarbonylamino and n-hexoxycarbonylamino.

Alkanoylamino illustratively and preferably represents acetylamino and ethylcarbonylamino.

Cycloalkyl per se and in cycloalkylamino and in cycloalkylcarbonyl represents a cycloalkyl group having generally 3 to 8 and preferably 5 to 7 carbon atoms, illustratively and preferably representing cyclopropyl, cyclobutyl, cyclopentyl, cyclohexyl and cycloheptyl.

Cycloalkylamino represents a cycloalkylamino radical having one or two (independently selected) cycloalkyl substituents, illustratively and preferably representing cyclopropylamino, cyclobutylamino, cyclopentylamino, cyclohexylamino and cycloheptylamino.

Cycloalkylcarbonyl illustratively and preferably represents cyclopropylcarbonyl, cyclobutylcarbonyl, cyclopentylcarbonyl, cyclohexylcarbonyl and cycloheptylcarbonyl.

Aryl per se and in arylamino and in arylcarbonyl represents a mono- to tricyclic aromatic carbocyclic radical having generally 6 to 14 carbon atoms, illustratively and preferably representing phenyl, naphthyl and phenanthrenyl.

Arylamino represents an arylamino radical having one or two (independently selected) aryl substituents, illustratively and preferably representing phenylamino, diphenylamino and naphthylamino.

Arylcarbonyl illustratively and preferably represents phenylcarbonyl and naphthylcarbonyl.

Heteroaryl per se and in heteroarylamino and heteroarylcarbonyl represents an aromatic mono- or bicyclic radical having generally 5 to 10 and preferably 5 or 6 ring atoms and up to 5 and preferably up to 4 hetero atoms selected from the group consisting of S, O and N, illustratively and preferably representing thienyl, furyl, pyrrolyl, thiazolyl, oxazolyl, imidazolyl, pyridyl, pyrimidyl, pyridazinyl, indolyl, indazolyl, benzofuranyl, benzothiophenyl, quinolinyl, isoquinolinyl.

Heteroarylamino represents an heteroarylamino radical having one or two (independently selected) heteroaryl substituents, illustratively and preferably representing thienylamino, furylamino, pyrrolylamino, thiazolylamino, oxazolylamino, imidazolyl-amino, pyridylamino, pyrimidylamino, pyridazinylamino, indolylamino, indazolylamino, benzofuranylamino, benzothiophenylamino, quinolinyl-amino, isoquinolinylamino.

Heteroarylcarbonyl illustratively and preferably represents thienylcarbonyl, furylcarbonyl, pyrrolylcarbonyl, thiazolylcarbonyl, oxazolylcarbonyl, imidazolylcarbonyl, pyridylcarbonyl, pyrimidylcarbonyl, pyridazinylcarbonyl, indolylcarbonyl, indazolylcarbonyl, benzofuranylcarbonyl, benzothiophenylcarbonyl, quinolinylcarbonyl, isoquinolinylcarbonyl.

Heterocyclyl per se and in heterocyclylcarbonyl represents a mono- or polycyclic, preferably mono- or bicyclic, nonaromatic heterocyclic radical having generally 4 to 10 and preferably 5 to 8 ring atoms and up to 3 and preferably up to 2 hetero atoms and/or hetero groups selected from the group consisting of N, O, S, SO and SO₂. The heterocyclyl radicals can be saturated or partially unsaturated. Preference is given to 5- to 8-membered monocyclic saturated heterocyclyl radicals having up to two hetero atoms selected from the group consisting of O, N and S, such as illustratively and preferably tetrahydrofuran-2-yl, pyrrolidin-2-yl, pyrrolidin-3-yl, pyrrolinyl, piperidinyl, morpholinyl, perhydroazepinyl.

Heterocyclylcarbonyl illustratively and preferably represents tetrahydrofuran-2-carbonyl, pyrrolidine-2-carbonyl, pyrrolidine-3-carbonyl, pyrrolinecarbonyl, piperidinecarbonyl, morpholinecarbonyl, perhydroazepinecarbonyl.

Halogen represents fluorine, chlorine, bromine and iodine.

### EMBODIMENT OF INVENTION

The compound of the formula (I) of the present invention can be, but not limited to be, prepared by combining various known methods. In some embodiments, one or more of the substituents, such as amino group, carboxyl group, and hydroxyl group of the compounds used as starting materials or intermediates are advantageously protected by a protecting group known to those skilled in the art. Examples of the protecting groups are described in "Protective Groups in Organic Synthesis (3rd Edition)" by Greene and Wuts, John Wiley and Sons, New York 1999.

The compound of the formula (I) of the present invention can be, but not limited to be, prepared by the Method [A] and [B] below.

### [Method A]

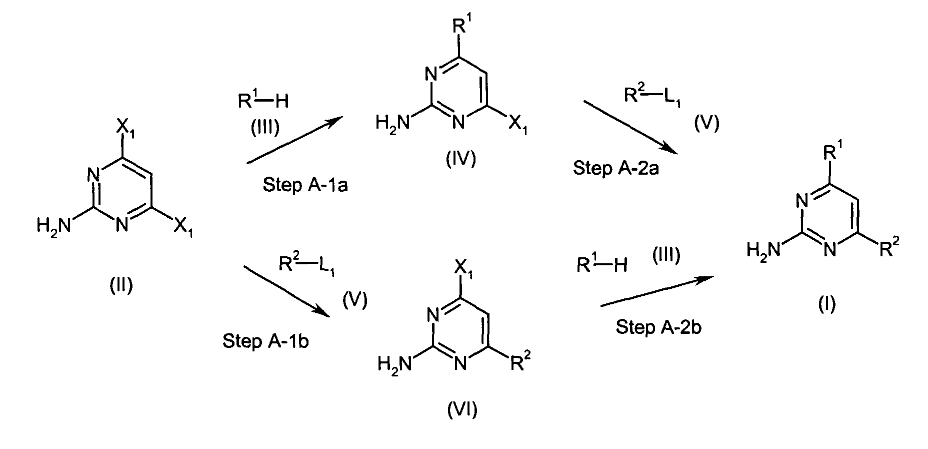

The compound of formula (I) (wherein R¹ and R² are the same as defined above) can be prepared, for example, in two steps.

In Step A-1a, compound of formula (IV) (wherein R¹ is the same as defined above and X₁ represents a leaving group including, for instance, halogen atom such as chlorine or bromine atom.) can be prepared by the reaction of the compound of formula (II) (wherein X₁ is the same as defined above.) with compound of formula (III) (wherein R¹ is the same as defined above).

The reaction may be carried out without solvent or in a solvent including, for instance, halogenated hydrocarbons such as dichloromethane, chloroform and 1,2-dichloroethane; ethers such as diethyl ether, isopropyl ether, dioxane and tetrahydrofuran (THF) and 1,2-dimethoxyethane; aromatic hydrocarbons such as benzene, toluene and xylene; nitriles such as acetonitrile; amides such as N, N-dimethylformamide (DMF), N, N-dimethylacetamide (DMAC) and N-methylpyrrolidone (NMP); urea such as 1,3-dimethyl-2-imidazolidinone (DMI); sulfoxides such as dimethylsulfoxide (DMSO); and others. Optionally, two or more of the solvents selected from the listed above can be mixed and used.

The reaction temperature can be optionally set depending on the compounds to be reacted. The reaction temperature is usually, but not limited to, about 0°C to 200°C and preferably about 20°C to 150°C. The reaction may be conducted for, usually, 10 minutes to 48 hours and preferably 30minutes to 24 hours.

The reaction can be advantageously carried out in the presence of a base including, for instance, organic amines such as pyridine, triethylamine, N,N-diisopropylethylamine, dimethylaniline, diethylaniline, and others.

In Step A-2a, compound of formula (I) (wherein R¹ and R² are the same as defined above) can be prepared by the reaction of compound of formula (IV) (wherein R¹ and X₁ are the same as defined above) with compound of formula (V) (wherein R² is the same as defined above and L₁ represents metal group including, for instance, organoborane group such as boronic acid and dimethoxy boryl; organostannyl group such as tributyl stannyl, and the like.)

The reaction can be advantageously carried out in the presence of a palladium catalyst such as tetrakis(triphenylphosphine)palladium.

The reaction can be advantageously carried out in the presence of a base including, for instance, cesium carbonate, sodium carbonate and potassium carbonate, and the like.

The reaction may be carried out in a solvent including, for instance, ethers such as diethyl ether, isopropyl ether, dioxane and tetrahydrofuran (THF) and 1,2-dimethoxyethane; aromatic hydrocarbons such as benzene, toluene and xylene; amides such as N, N-dimethylformamide (DMF), N, N-dimethylacetamide and N-methylpyrrolidone; sulfoxides such as dimethylsulfoxide (DMSO); alcohols such as methanol, ethanol, 1-propanol, isopropanol and tert-butanol and others. Optionally, two or more of the solvents selected from the listed above can be mixed and used.

The reaction temperature can be optionally set depending on the compounds to be reacted. The reaction temperature is usually, but not limited to, about 20°C to 120°C. The reaction may be conducted for, usually, 30 minutes to 48 hours and preferably 1 to 24 hours.

The compound of formula (I) (wherein R¹ and R² are the same as defined above) can also be prepared by the following procedures.

In Step A-1b, compound of formula (VI) (wherein R² and X₁ are the same as defined above) can be prepared by the reaction of compound of formula (II) (wherein X₁ is the same as defined above) with compound of formula (V) (wherein L₁ and R² are the same as defined above) in a similar manner described in Step A-2a for the preparation of compound of the formula (I).

In Step A-2b, compound of formula (I) (wherein R¹ and R² are the same as defined above) can be prepared by the reaction of compound of formula (VI) (wherein R² and X₁ are the same as defined above) with compound of formula (III) (wherein R¹ is the same as defined above) in a similar manner described in Step A-1a for the preparation of compound of the formula (IV).

The compound of formula (II), (III) and (V) can be prepared by the use of known techniques or are commercially available.

### Preparation of the compound of formula (VI)

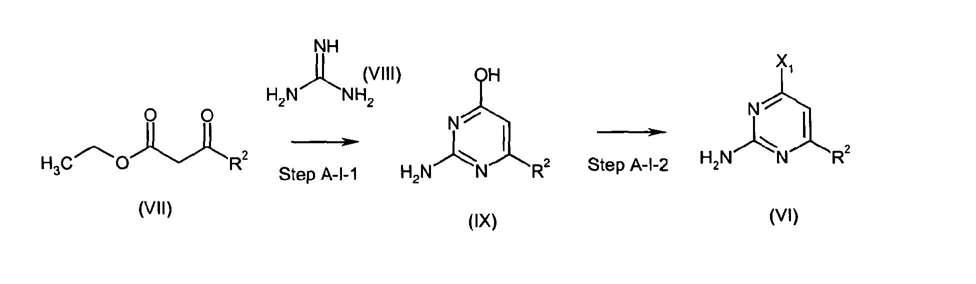

The compound of formula (VI) (wherein R² and X₁ are the same as defined above) can alternatively be prepared by the following procedures.

In Step A-I-1, compound of formula (IX) (wherein R² is the same as defined above) can be prepared by the reaction of compound of formula (VII) (wherein R² is the same as defined above) with compound of formula (VIII) or any salt form of (VIII).

The reaction can be advantageously carried out in the presence of a base including, for instance, sodium hydride, potassium hydride, sodium methoxide, sodium ethoxide, sodium hydroxide or potassium hydroxide, and the like.

The reaction may be carried out in a solvent including, for instance, alcohols such as methanol, ethanol, 1-propanol, isopropanol and tert-butanol and others. Optionally, two or more of the solvents selected from the listed above can be mixed and used.

The reaction temperature can be optionally set depending on the compounds to be reacted. The reaction temperature is usually, but not limited to, about 20°C to 120°C. The reaction may be conducted for, usually, 30 minutes to 48 hours and preferably 1 to 24 hours.

In Step A-I-2, compound of the formula (VI) (wherein R² and X₁ are the same as defined above) can be prepared by the reaction of compound of formula (IX) (wherein R² is the same as defined above) with an appropriate halogenating reagent including, for instance, POCl₃, POBr₃, PCl₅, and the like.

The reaction may be carried out without solvent or in a solvent including, for instance, halogenated hydrocarbons such as dichloromethane, chloroform and 1,2-dichloroethane; aromatic hydrocarbons such as benzene, and others.

The reaction can be advantageously conducted in the presence of a base, including, for instance, such as pyridine, triethylamine and N,N-diisopropylethylamine, dimethylaniline, diethylaniline, and others.

The reaction temperature can be optionally set depending on the compounds to be reacted. The reaction temperature is usually, but not limited to, about 0°C to 200°C and preferably about 20°C to 150°C. The reaction may be conducted for, usually, 10 minutes to 48 hours and preferably 30minutes to 24 hours.

The compound of formula (VII), (VIII) or any salt form of (VIII) can be prepared by the use of known techniques or are commercially available.

### [Method B]

The compound of formula (I) (wherein R¹ and R² are the same as defined above) can also be prepared by the following procedures.

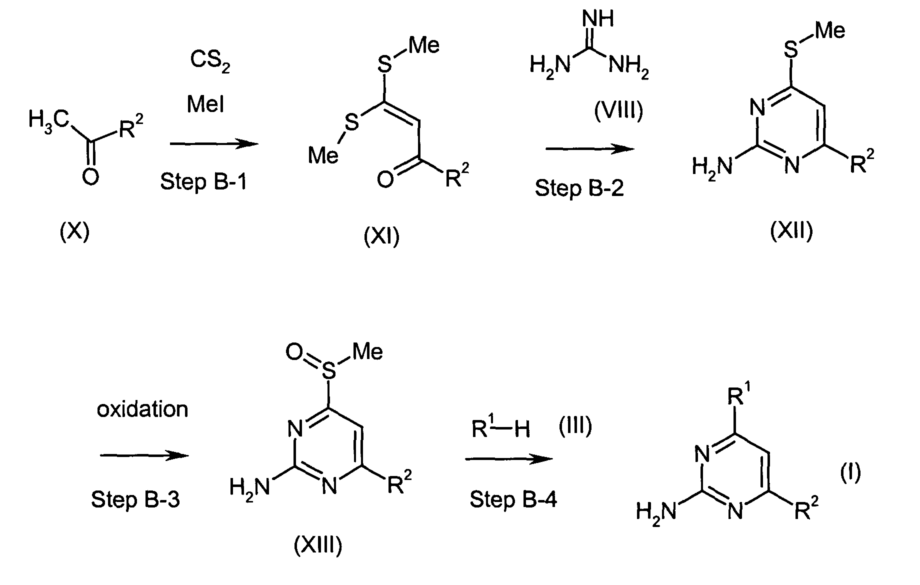

In the Step-B-1, compound of formula (XI) (wherein R² is the same as defined above) can be prepared by the reaction of the compound of formula (X) (wherein R² is the same as defined above) with methyl iodide and carbon disulfide in the presence of a base including, for instance, sodium hydride, potassium hydride, sodium methoxide or sodium ethoxide, sodium hydroxide or potassium hydroxide.

The reaction can be carried out in a solvent including, for instance, halogenated hydrocarbons such as dichloromethane, chloroform and 1,2-dichloroethane; ethers such as diethyl ether, isopropyl ether, dioxane and tetrahydrofuran (THF) and 1,2-dimethoxyethane; aromatic hydrocarbons such as benzene, toluene and xylene; dimethylformamide (DMF), dimethylacetamide (DMAC), 1,3-dimethyl-3,4,5,6-tetrahydro-2(1H)-pyrimidinone (DMPU), 1,3-dimethyl-2-imidazolidinone (DMI), N-methylpyrrolidinone (NMP), sulfoxides such as dimethylsulfoxide (DMSO), and others. Optionally, two or more of the solvents selected from the listed above can be mixed and used.

The reaction temperature is usually, but not limited to, about 0°C to 200°C and preferably about 20°C to 150°C. The reaction may be conducted for, usually, 30 minutes to 48 hours and preferably 2 hours to 24 hours.

In the Step-B-2, compound of formula (XII) (wherein R² is the same as defined above) can be prepared by the reaction of compound of formula (XI) (wherein R² is the same as defined above) with the compound of formula (VIII) or any salt form of (VIII).

The reaction can be advantageously carried out in the presence of a base including, for instance, sodium hydride, potassium hydride, sodium methoxide, sodium ethoxide, sodium hydroxide or potassium hydroxide, and the like.

The reaction can be carried out in a solvent including, for instance, ethers such as diethyl ether, isopropyl ether, dioxane and tetrahydrofuran (THF) and 1,2-dimethoxyethane; aromatic hydrocarbons such as benzene, toluene and xylene; dimethylformamide (DMF), dimethylacetamide(DMAC), 1,3-dimethyl-3,4,5,6-tetrahydro-2(1H)-pyrimidinone (DMPU), 1,3-dimethyl-2-imidazolidinone (DMI), N-methylpyrrolidinone (NMP), sulfoxides such as dimethylsulfoxide (DMSO), alcohols such as methanol, ethanol, 1-propanol, isopropanol and tert-butanol, water and others. Optionally, two or more of the solvents selected from the listed above can be mixed and used.

The reaction temperature can be optionally set depending on the compounds to be reacted. The reaction temperature is usually, but not limited to, about 20°C to 120°C. The reaction may be conducted for, usually, 30 minutes to 48 hours and preferably 1 to 24 hours.

In the Step-B-3, compound of formula (XIII) (wherein R² is the same as defined above) can be prepared by the oxidation of sulfur atom of compound of formula (XII) (wherein R² is the same as defined above).

The oxidation reaction can be performed using a reagent, such as hydrogen peroxide, sodium periodate, m-chloroperbenzoic acid (m-CPBA), potassium permanganate and others in the absence or presence of catalyst, such as catalytic ruthenium trichloride.

The reaction can be carried out in solvent including, for instance, water, halogenated hydrocarbons such as, methylene chloride, carbon tetrachloride, chlorobenzene, dichloromethane, chloroform and 1,2-dichloroethane; ethers such as diethyl ether, isopropyl ether, dioxane and tetrahydrofuran (THF) and 1,2-dimethoxyethane; aromatic hydrocarbons such as benzene, toluene and xylene; nitriles such as acetonitrile; amides such as N, N-dimethylformamide (DMF), N, N-dimethylacetamide and N-methylpyrrolidone; and others. Optionally, two or more of the solvents selected from the listed above can be mixed and used.

The reaction temperature is usually, but not limited to about -10°C to 200°C, and preferably about 10°C to 50°C. The reaction may be carried out for, usually, 30 minutes to 48 hours and preferably 1 hour to 20 hours.

In Step B-4, compound of formula (I) (wherein R¹ and R² are the same as defined above) can be prepared by the reaction of compound of formula (XIII) (wherein R² is the same as defined above) with compound of formula (III) (wherein R¹ is the same as defined above).

The reaction may be carried out without solvent or in a solvent including, for instance, halogenated hydrocarbons such as dichloromethane, chloroform and 1,2-dichloroethane; ethers such as diethyl ether, isopropyl ether, dioxane and tetrahydrofuran (THF) and 1,2-dimethoxyethane; aromatic hydrocarbons such as benzene, toluene and xylene; nitriles such as acetonitrile; amides such as N, N-dimethylformamide (DMF), N, N-dimethylacetamide (DMAC) and N-methylpyrrolidone (NMP); urea such as 1,3-dimethyl-2-imidazolidinone (DMI); sulfoxides such as dimethylsulfoxide (DMSO); and others. Optionally, two or more of the solvents selected from the listed above can be mixed and used.

The reaction temperature can be optionally set depending on the compounds to be reacted. The reaction temperature is usually, but not limited to, about 0°C to 200°C and preferably about 20°C to 150°C. The reaction may be conducted for, usually, 10 minutes to 48 hours and preferably 30minutes to 24 hours.

The compound of formula (X) can be prepared by the use of known techniques or are commercially available.

When the compound shown by the formula (I) or a salt thereof has an asymmetric carbon(s) in the structure, their optically active compounds and racemic mixtures are also included in the scope of the present invention.

Typical salts of the compound shown by the formula (I) include salts prepared by the reaction of the compound of the present invention with a mineral or organic acid, or an organic or inorganic base. Such salts are known as acid addition and base addition salts, respectively.

Acids to form acid addition salts include inorganic acids such as, without limitation, sulfuric acid, phosphoric acid, hydrochloric acid, hydrobromic acid, hydroiodic acid and the like, and organic acids, such as, without limitation, p-toluenesulfonic acid, methanesulfonic acid, oxalic acid, p-bromophenylsulfonic acid, carbonic acid, succinic acid, citric acid, benzoic acid, acetic acid, and the like.

Base addition salts include those derived from inorganic bases, such as, without limitation, ammonium hydroxide, alkaline metal hydroxide, alkaline earth metal hydroxides, carbonates, bicarbonates, and the like, and organic bases, such as, without limitation, ethanolamine, triethylamine, tri(hydroxymethyl)aminomethane, and the like. Examples of inorganic bases include, sodium hydroxide, potassium hydroxide, potassium carbonate, sodium carbonate, sodium bicarbonate, potassium bicarbonate, calcium hydroxide, calcium carbonate, and the like.

The compound of the present invention or a salts thereof, depending on its substituents, may be modified to form lower alkylesters or known other esters; and/or hydrates or other solvates. Those esters, hydrates, and solvates are included in the scope of the present invention.

The compound of the present invention may be administered in oral forms, such as, without limitation normal and enteric coated tablets, capsules, pills, powders, granules, elixirs, tinctures, solution, suspensions, syrups, solid and liquid aerosols and emulsions. They may also be administered in parenteral forms, such as, without limitation, intravenous, intraperitoneal, subcutaneous, intramuscular, and the like forms, well-known to those of ordinary skill in the pharmaceutical arts. The compounds of the present invention can be administered in intranasal form via topical use of suitable intranasal vehicles, or via transdermal routes, using transdermal delivery systems well-known to those of ordinary skilled in the art.

The dosage regimen with the use of the compounds of the present invention is selected by one of ordinary skill in the arts, in view of a variety of factors, including, without limitation, age, weight, sex, and medical condition of the recipient, the severity of the condition to be treated, the route of administration, the level of metabolic and excretory function of the recipient, the dosage form employed, the particular compound and salt thereof employed.

The compounds of the present invention are preferably formulated prior to administration together with one or more pharmaceutically-acceptable excipients. Excipients are inert substances such as, without limitation carriers, diluents, flavoring agents, sweeteners, lubricants, solubilizers, suspending agents, binders, tablet disintegrating agents and encapsulating material.

Yet another embodiment of the present invention is pharmaceutical formulation comprising a compound of the invention and one or more pharmaceutically-acceptable excipients that are compatible with the other ingredients of the formulation and not deleterious to the recipient thereof. Pharmaceutical formulations of the invention are prepared by combining a therapeutically effective amount of the compounds of the invention together with one or more pharmaceutically-acceptable excipients. In making the compositions of the present invention, the active ingredient may be mixed with a diluent, or enclosed within a carrier, which may be in the form of a capsule, sachet, paper, or other container. The carrier may serve as a diluent, which may be solid, semi-solid, or liquid material which acts as a vehicle, or can be in the form of tablets, pills, powders, lozenges, elixirs, suspensions, emulsions, solutions, syrups, aerosols, ointments, containing, for example, up to 10% by weight of the active compound, soft and hard gelatin capsules, suppositories, sterile injectable solutions and sterile packaged powders.

For oral administration, the active ingredient may be combined with an oral, and non-toxic, pharmaceutically-acceptable carrier, such as, without limitation, lactose, starch, sucrose, glucose, sodium carbonate, mannitol, sorbitol, calcium carbonate, calcium phosphate, calcium sulfate, methyl cellulose, and the like; together with, optionally, disintegrating agents, such as, without limitation, maize, starch, methyl cellulose, agar bentonite, xanthan gum, alginic acid, and the like; and optionally, binding agents, for example, without limitation, gelatin, natural sugars, beta-lactose, corn sweeteners, natural and synthetic gums, acacia, tragacanth, sodium alginate, carboxymethylcellulose, polyethylene glycol, waxes, and the like; and, optionally, lubricating agents, for example, without limitation, magnesium stearate, sodium stearate, stearic acid, sodium oleate, sodium benzoate, sodium acetate, sodium chloride, talc, and the like.

In powder forms, the carrier may be a finely divided solid which is in admixture with the finely divided active ingredient. The active ingredient may be mixed with a carrier having binding properties in suitable proportions and compacted in the shape and size desired to produce tablets. The powders and tablets preferably contain from about 1 to about 99 weight percent of the active ingredient which is the novel composition of the present invention. Suitable solid carriers are magnesium carboxymethyl cellulose, low melting waxes, and cocoa butter.

Sterile liquid formulations include suspensions, emulsions, syrups and elixirs. The active ingredient can be dissolved or suspended in a pharmaceutically acceptable carrier, such as sterile water, sterile organic solvent, or a mixture of both sterile water and a sterile organic solvent.

The active ingredient can also be dissolved in a suitable organic solvent, for example, aqueous propylene glycol. Other compositions can be made by dispersing the finely divided active ingredient in aqueous starch or sodium carboxymethyl cellulose solution or in a suitable oil.

The formulation may be in unit dosage form, which is a physically discrete unit containing a unit dose, suitable for administration in human or other mammals. A unit dosage form can be a capsule or tablets, or a number of capsules or tablets. A "unit dose" is a predetermined quantity of the active compound of the present invention, calculated to produce the desired therapeutic effect, in association with one or more excipients. The quantity of active ingredient in a unit dose may be varied or adjusted from about 0.1 to about 1000 milligrams or more according to the particular treatment involved.

Typical oral dosages of the present invention, when used for the indicated effects, will range from about 0.01mg/kg/day to about 100mg/kg/day, preferably from 0.1 mg/kg/day to 30 mg/kg/day, and most preferably from about 0.5 mg/kg/day to about 10 mg/kg/day. In case of parenteral administration, it has generally proven advantageous to administer quantities of about 0.001 to 100 mg/kg/day, preferably from 0.01 mg/kg/day to 1mg/kg/day. The compounds of the present invention may be administered in a single daily dose, or the total daily dose may be administered in divided doses, two, three, or more times per day. Where delivery is via transdermal forms, of course, administration is continuous.

### EXAMPLES

The present invention will be described in detail below in the form of examples, but they should by no means be construed as defining the metes and bounds of the present invention.

In the examples below, all quantitative data, if not stated otherwise, relate to percentages by weight.

¹H NMR spectra were recorded using either Bruker DRX-300 (300 MHz for ¹H) spectrometer or Brucker 500 UltraShieled™ (500 MHz for 1H). Chemical shifts are reported in parts per million (ppm) with tetramethylsilane (TMS) as an internal standard at zero ppm. Coupling constant (J) are given in hertz and the abbreviations s, d, t, q, m, and br refer to singlet, doublet, triplet, quartet, multiplet, and broad, respectively. The mass determinations were carried out by MAT95 (Finnigan MAT).

Liquid Chromatography - Mass spectroscopy (LC-MS) data were recorded on a Micromass Platform LC with Shimadzu Phenomenex ODS column(4.6 mm φ X 30 mm) flushing a mixture of acetonitrile-water (9:1 to 1:9) at 1 ml/min of the flow rate. Mass spectra were obtained using electrospray (ES) ionization techniques (Micromass Platform LC). TLC was performed on a precoated silica gel plate (Merck silica gel 60 F-254). Silica gel (WAKO-gel C-200 (75-150 µm)) was used for all column chromatography separations. All chemicals were reagent grade and were purchased from Sigma-Aldrich, Wako pure chemical industries, Ltd., Tokyo kasei kogyo Co., Ltd., Nacalai tesque, Inc., Watanabe Chemical Ind. Ltd., Maybridge plc, Lancaster Synthesis Ltd., Merck KgaA, Kanto Chemical Co., Ltd.

The effects of the compounds of the present invention were examined by the following assays and pharmacological tests.

### [Calcium Mobilization in CHO cells transfected with human H4] (Assay1)

A fusion construct consisting of the n-terminal part of H₄ and the C-terminal part of the G protein Gα 6 was transfected into CHO cells containing the aequorin-controlled luciferase gene (CHOAQLuci cells). Recombinant clones showing aequorin luminescence after the addition of 1 µM histamine were isolated. Starting with three independent primary cell clones three sets of tertiary clones were generated by two successive rounds of limiting dilution. Analysis of the primary clones as well as each subcloning by limiting dilution was monitored by "clone-pool analysis" comparing H₄-transfected to mock-transfected (expression vector without H₄ receptor cDNA) clones.

CHO-H₄ cells were seeded at 2 x 10⁴ cells per well in Biocoat poly-D-Lysine 96 well black/clear plates (BECTON DICKINSON, Franklin Lakes, US) in D-MEM/F12 medium supplemented with 10% FCS and 400 µg/ml Geneticin (Invitrogen GIBCO, Carlsbad, US) and 2 mM butylrate for O/N. The medium was removed and cells were loaded with 1 µM Fluo-3 (Molecular Probes, Eugene, USA) in loading solution (0.1% BSA, 20mM HEPES Hank's Buffer, 1mM probinecid) for 1 h. The loading buffer was removed and exchanged for fresh loading buffer. Cells were pretreated with various concentrations of test compounds and subjected to calcium mobilization measurement induced by 100 nM histamine by using FDSS6000.

### [Histamine H4 binding assay] (Assay 2)

Membrane fraction was prepared from CHO-H₄ stable cells treated with 5 mM butyrate for O/N. 20 nM ³H-histamine binding to membrane was performed in 50 mM Tris (pH 7.5 ), 5 mM EDTA for 60 min in the presence of test compounds. Non-specific binding was determined in the presence of 20 µM histamine. B/F separation was performed by filtration using MAFB NOB plate ( Millipore, Tokyo, JAPAN) with 3 times wash by 50 mM Tris (pH7.5 ), 0.5 mM EDTA.

### [Migration assay] (Assay 3)

Bone marrow-derived mast cells (BMMC) were generated from the femural bone marrow of 6-8 week old male mice and maintained in RPMI1640 medium (Nacalai Tesque, Kyoto Japan) supplemented with 10% fetal bovine serum (FBS; JRH Bioscience, Lenex, KS, US), 100 units/ml penicilline-streptpmycine (GIBCO BRL, Rockville, MD, US) and 10 ng/ml recombinant murine IL-3 (Pepro Tech EC Ltd., London, UK) at a density of 1x10⁶ cells/ml. Prior to experimentation, BMMC were harvested, washed twice in PBS and further used as indicated. Purity of mast cells was >95% as asessed by Toluidine blue staining or FACS analysis of cell surface expression of c-kit and FcERI.

Transwell plates (Costar, NY, USA) with a pore size of 8µm were coated with 100 µl of 100 ng / ml human fibronectin for 2h at room temperature. After removal of fibronectin 600 µl of RPMI containing 10% FCS in the presence of histamine were added to the bottom chamber. Subsequently, bone marrow-derived mast cells (2 x 10⁵ / well in 100 µl ) pre-treated with antagonists or test compounds were added to the top chambers. The plates were incubated for 2 h at 37C degrees. Transwells were removed and the number of cells in the bottom chamber was counted by flow cytometer.

### [Zymosan-induced pleurisy in mice] (Assay 4)

Mice (Balb/c, 7-8 weeks old, SPF, Charles River Inc.) received a single intrapleural injection with 0.2 ml zymosan (100 µg/mouse) under anesthetization with ether. Test compounds (thioperamide, dexamethasone, and vehicle (10 % cremophor in PBS)) were administered p.o. (0.2 ml/head) 60 minutes prior to zymosan injection. Four hours after injection, mice were sacrificed and pleural fluid was collected by washing the pleural cavity twice with 2 ml PBS. The cell suspension was diluted to one tenth in Turk's stain solution (Nacalai Tesque). The number of total cells in the sample was counted under the microscope using a hemocytometer. Cytospin specimens were stained with May-Gruenwald's (Merck, Darmstadt, Germany) and Giemsa's solution (Merck) for leukocyte typing. The distribution of each cell population (neutrophils, eosinophils, macrophages, lymphocytes and others) was counted under microscopy by counting more than 200 cells.

### Statistics

Unless otherwise stated, data are expressed as means ± SD of at least three independent experiments. Statistical significance was determined using the unpaired Student's *t*-test if applicable or with the Dunnett's or Welch test if variances were non-homogeneous using commercially available statistic software (GraphPad Software, San Diego, CA).

Assay results in Assay 1 are shown in Examples and tables of the Examples below. The data corresponds to the compounds as yielded by solid phase synthesis and thus to levels of purity of about 40 to 90%. For practical reasons, the compounds are grouped in four classes of activity as follows:
A IC₅₀ 20nM < B IC₅₀ 100nM C < IC₅₀ 500nM D

The compounds of the present invention also show excellent selectivity, and potent activity in Assays 2 - 4 described above.

Z used in Melting point in the following section indicates decomposition.

### Preparation of compounds

### Example 1

### 4-[3-(Methylamino)pyrrolidin-1-yl]-6-phenylpyrimidin-2-amine trihydrochloride

### 1) tert-Butyl [1-(2-amino-6-chloropyrimidin-4-yl)pyrrolidin-3-yl]methylcarbamate

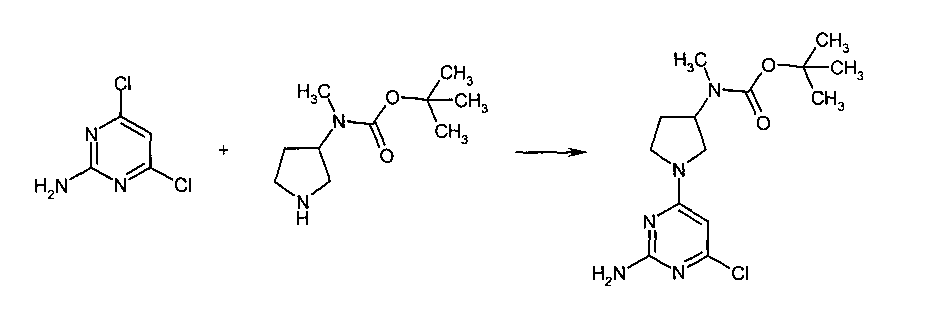

A mixture of 2-amino-4,6-dichloropyrimidine (1.00 g), tert-butyl methyl(pyrrolidin-3-yl)carbamate (1.22 g), and *N*,*N*-diisopropylethylamine (1.59 ml) in EtOH (6.0 ml) was refluxed for 3 h. Silica gel (ca. 2 g) was added, and the mixture was concentrated in vacuo. The residual solid was applied to silica gel column chromatography (hexane/ethyl acetate 3/2 to 1/1) to give tert-butyl [1-(2-amino-6-chloropyrimidin-4-yl)pyrrolidin-3-yl]methylcarbamate (1.33 g, 66% yield) as a white solid.
¹H NMR (500 MHz, CDCl₃) δ 1.48 (s, 9H), 2.07 (br s, 2H), 2.15 (br s, 2H), 2.79 (s, 3H), 3.1-4.0 (br, 4H), 4.83 (br s, 3H), 5.77 (s, 1H).

### 2) tert-Butyl [1-(2-amino-6-phenylpyrimidin-4-yl)pyrrolidin-3-yl]methylcarbamate

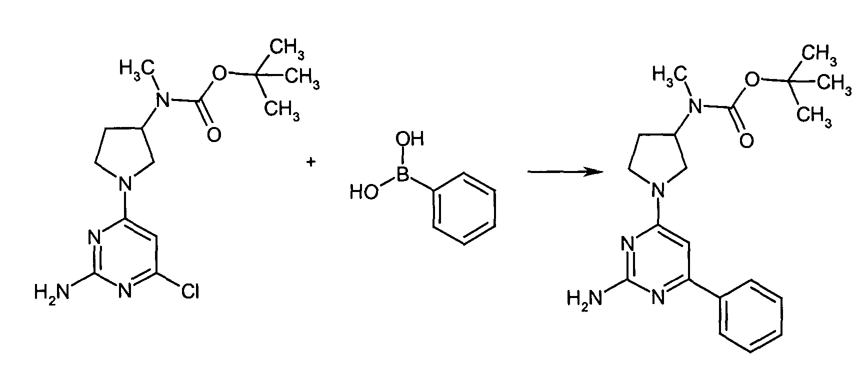

To a mixture of give tert-butyl [1-(2-amino-6-chloropyrimidin-4-yl)-pyrrolidin-3-yl]methylcarbamate (0.60 g), Pd(PPh₃)₄ (0.126 g) in DME (4 ml) was added phenylboronic acid immediately followed by aqueous Na₂CO₃ (2 M, 2 ml). The mixture was flushed with Ar, and the reaction mixture was then refluxed for 16 h. The mixture was cooled to room temperature, and then partitioned between ethyl acetate and water. The organic layer was washed with brine, dried over sodium sulfate, and evapotated. Purification by column chromatography (NH-DM1020, hexane/ethyl acetate 2:1 to 1:2) gave tert-butyl [1-(2-amino-6-phenylpyrimidin-4-yl)pyrrolidin-3-yl]methylcarbamate (0.603 g, 89% yield) as a colorless foam.
¹H NMR (500 MHz, CDCl₃) δ 1.49 (s, 9H), 2.02-2.23 (m, 2H), 2.81 (s, 3H), 3.39 (br s, 1H), 3.46 (q, *J =* 8.2 Hz, 1 H), 3.72 (br s, 2H), 4.79 (s, 2H), 4.87 (br s, 1H), 6.14 (s, 1H), 7.40-7.44 (m, 3H), 7.89-7.93 (m, 2H).

### 3) 4-[3-(Methylamino)pyrrolidin-1-yl]-6-phenylpyrimidin-2-amine trihydrochloride

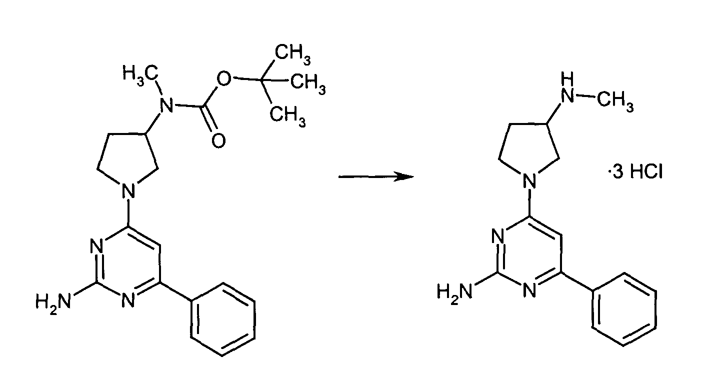

To a solution of tert-butyl [1-(2-amino-6-phenylpyrimidin-4-yl)pyrrolidin-3-yl]methylcarbamate (0.603 g) in EtOH (1.0 ml) was added aqueous 6 N HCl solution (1.0 ml), and the mixture was stirred at 100°C for 10 min. The reaction mixture was diluted with EtOH, and then concentrated in vacuo. The residue was triturated with EtOH-AcOEt to give 4-[3-(methylamino)-pyrrolidin-1-yl]-6-phenylpyrimidin-2-amine trihydrochloride (0.465 g, 75% yield) as a white solid.
¹H NMR (500 MHz, DMSO-*d*_{*6*}) as a equilibrium mixture: δ 2.24-2.47 (m, 2H), 2.59 and 2.61 (each s, 3H), 3.64-3.80 (m, 1H), 3.80-4.00 (m, 4H), 6.65 and 6.67 (each s, 1H), 7.58-7.69 (m, 3H), 7.70-8.80 (br, 3H), 8.07 (br s, 2H), 9.4-10.1 (m, 2H), 13.02 (br s, 1H).
Molecular weight: 378.73
Mass spectrometry: m/z 270 (MH⁺ - 3HCl).
Melting point (°C): 303 Z
Activity Class : A

### Example 2

### 3-{2-Amino-6-[3-(methylamino)pyrrolidin-1-yl]pyrimidin-4-yl}benzonitrile trihydrochloride

### 1) 3-[3,3-Bis(methylthio)acryloyl]benzonitrile

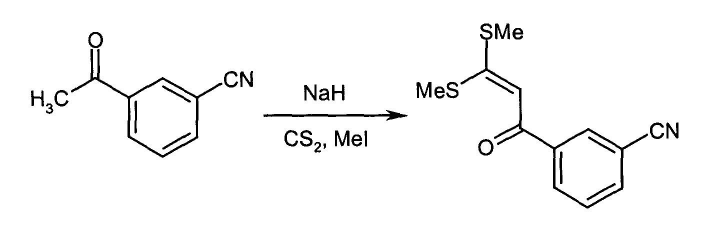

To a suspension of NaH (60% oil suspension, 2.76 g) in DMSO (25 ml) was added a solution of 3-acetylbenzonitrile (5.00 g) in DMSO (25 ml). CS₂ (2.07 ml) was added dropwise with external water bath cooling, and the mixture was stirred for 45 min. Methyl iodide (4.29 ml) was added dropwise with external water bath cooling, and the mixture was stirred at room temperature overnight. The reaction mixture was poured into water (300 ml), stirred for 10 min, and the resulting precipitate was collected by filtration and dried in vacuo to give 3-[3,3-bis(methylthio)acryloyl]benzonitrile (8.39 g, 98% yield) as a brown solid.
¹H NMR (500 MHz, DMSO-*d*_{*6*}) δ 2.51 (s, 3H), 2.71 (s, 3H), 6.90 (s, 1H), 7.71 (t, *J =* 7.8 Hz, 1H), 8.03 (dt, *J =* 1.4, 7.8 Hz, 1H), 8.25 (dt, *J =* 1.4, 7.8 Hz, 1H), 8.44 (t, *J=* 1.4 Hz, 1H).

### 2) 3-[2-Amino-6-(methylthio)pyrimidin-4-yl]benzonitrile

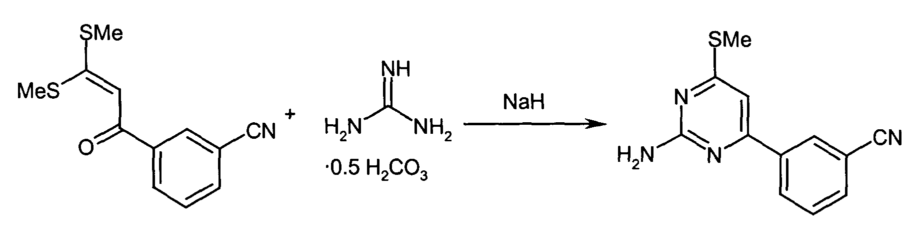

To a stirred suspension of NaH (60% oil suspension, 0.64 g) in DMF was added 3-[3,3-bis(methylthio)acryloyl]benzonitrile (4.00 g) by portions, and the mixture was stirred at room temperature for 10 min. Guanidine carbonate (3.47 g) was added by portions, and the mixture was stirred at 100°C for 3 h. The reaction mixture was poured into ice-cold water (300 ml), stirred for 0.5 h, and the resulting precipitate was collected by filtration. The crude product was purified by column chromatography (NH-DM1020, hexane/ethyl acetate = 3:1) and then recrystallized from diisopropyl ether-ethyl acetate to afford 3-[2-amino-6-(methylthio)pyrimidin-4-yl]benzonitrile (0.57 g, 15% yield) as a white solid.
¹H NMR (500 MHz, CDCl3) δ 2.55 (s, 3H), 5.09 (s, 2H), 6.92 (s, 1H), 7.56 (t, *J =* 7.9 Hz, 1H), 7.72 (d, *J =* 7.9 Hz, 1H), 8.16 (d, *J =* 7.9 Hz, 1H), 8.29 (s, 1H).

### 3) 3-[2-Amino-6-(methylsulfinyl)pyrimidin-4-yl]benzonitrile

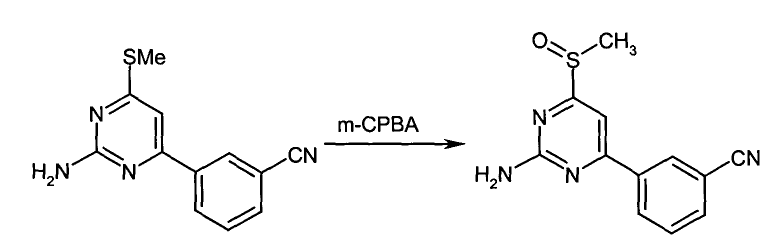

To a solution of 3-[2-amino-6-(methylthio)pyrimidin-4-yl]benzonitrile (0.50 g) in CH₂Cl₂ (10 ml) cooled to 0°C was added m-CPBA (77%, 0.55 g), and the mixture was stirred at 0°C for 1 h. The reaction mixture was quenched with saturated aqueous solution of NaHCO₃ (30 ml) containing Na₂S₂O₃·5H₂O (1 g), and the resulting precipitate was collected by filtration and dried in vacuo to give 3-[2-amino-6-(methylsulfinyl)pyrimidin-4-yl]benzonitrile (0.287 g, 54% yield) as a pale yellow solid.
¹H NMR (500 MHz, DMSO-*d*6) δ 2.85 (s, 3H), 7.32 (s, 2H), 7.61 (s, 1H), 7.75 (t, *J =* 7.9 Hz, 1H), 8.02 (d, *J =* 7.9 Hz, 1H), 8.44 (d, *J* = 7.9 Hz, 1H), 8.57 (s, 1H).

### 4) tert-Butyl {1-[2-amino-6-(3-cyanophenyl)pyrimidin-4-yl]pyrrolidin-3-yl}methylcarbamate

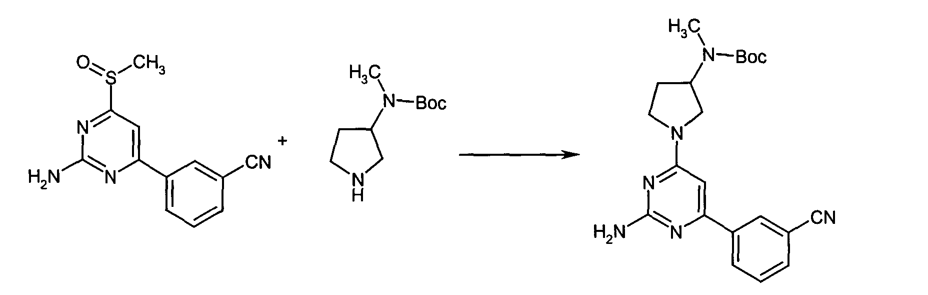

A mixture of 3-[2-amino-6-(methylsulfinyl)pyrimidin-4-yl]benzonitrile (0.12 g), tert-butyl methyl(pyrrolidin-3-yl)carbamate (0.14 g), and *N*,*N*-diisopropylethylamine (0.12 ml) in EtOH (2 ml) was refluxed for 16 h. The mixture was partitioned between ethyl acetate and water. The organic layer was washed with brine, dried over Na₂SO₄, and concentrated in vacuo. Purification by column chromatography (NH-DM1020, hexane/ethyl acetate = 1:1) gave tert-butyl {1-[2-amino-6-(3-cyanophenyl)pyrimidin-4-yl]-pyrrolidin-3-yl}methylcarbamate (0.059 g, 32% yield) as a colorless oil.
¹H NMR (500 MHz, CDCl₃) δ 1.49 (s, 9H), 2.05-2.25 (m, 2H), 2.82 (s, 3H), 3.40 (br s, 1h), 3.43-3.52 (m, 1H), 3.73 (br s, 1H), 4.7-5.0 (br, 1H), 4.84 (s, 2H), 6.12 (s, 1H), 7.53 (t, *J =* 7.9 Hz, 1H), 7.69 (dt, *J =* 1.4, 7.9 Hz, 1H), 8.16 (dt, *J =* 1.4, 7.9 Hz, 1H), 8.24 (t, *J =* 1.4 Hz, 1H).

### 5) 3-{2-Amino-6-[3-(methylamino)pyrrolidin-1-yl]pyrimidin-4-yl}benzonitrile trihydrochloride

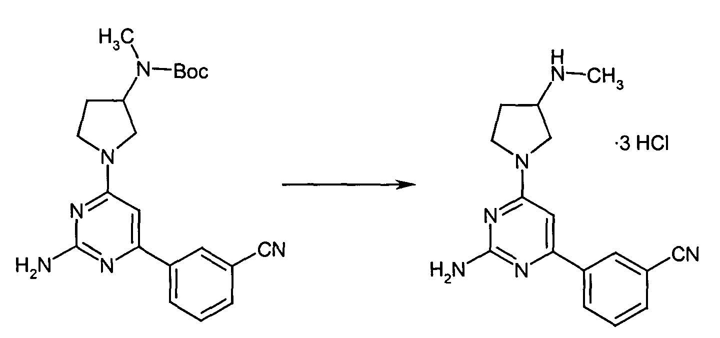

To a solution of tert-butyl {1-[2-amino-6-(3-cyanophenyl)pyrimidin-4-yl]-pyrrolidin-3-yl}methylcarbamate (0.059 g) in EtOH (1.0 ml) was added aqueous 1 N HCl solution (1.0 ml), and the mixture was stirred at 80°C for 2 h. The reaction mixture was diluted with EtOH, and then concentrated in vacuo. The residue was triturated with ethyl acetate to give 3-{2-amino-6-[3-(methylamino)pyrrolidin-1-yl]pyrimidin-4-yl}benzonitrile trihydrochloride (0.050 g, 83% yield) as a white solid.
¹H NMR (500 MHz, DMSO-*d*_{*6*}) *δ* 2.23-2.48 (m, 2H), 2.60 (s, 3H), 3.65-3.80 (m, 1H), 3.80-4.03 (m, 4H), 6.80 (s, 1H), 7.82 (t, *J =* 7.9 Hz, 1H), 8.11 (d, *J* = 7.9 Hz, 1H), 8.40 (br d, *J* = 7.9 Hz, 1H), 8.57 (s, 1H), 7.30-8.80 (br, 3H), 9.4-10.0 (m, 2H), 13.22 (br s, 1H).
Molecular weight: 403.74
Mass spectrometry: m/z 294 (MH⁺ - 3HCl).
Melting point (°C): 243 Z
Activity Class : B

In a similar manner as described in either Example 1 or Example 2 , compounds in Example 3 to 35 shown in Table 1 were synthesized.

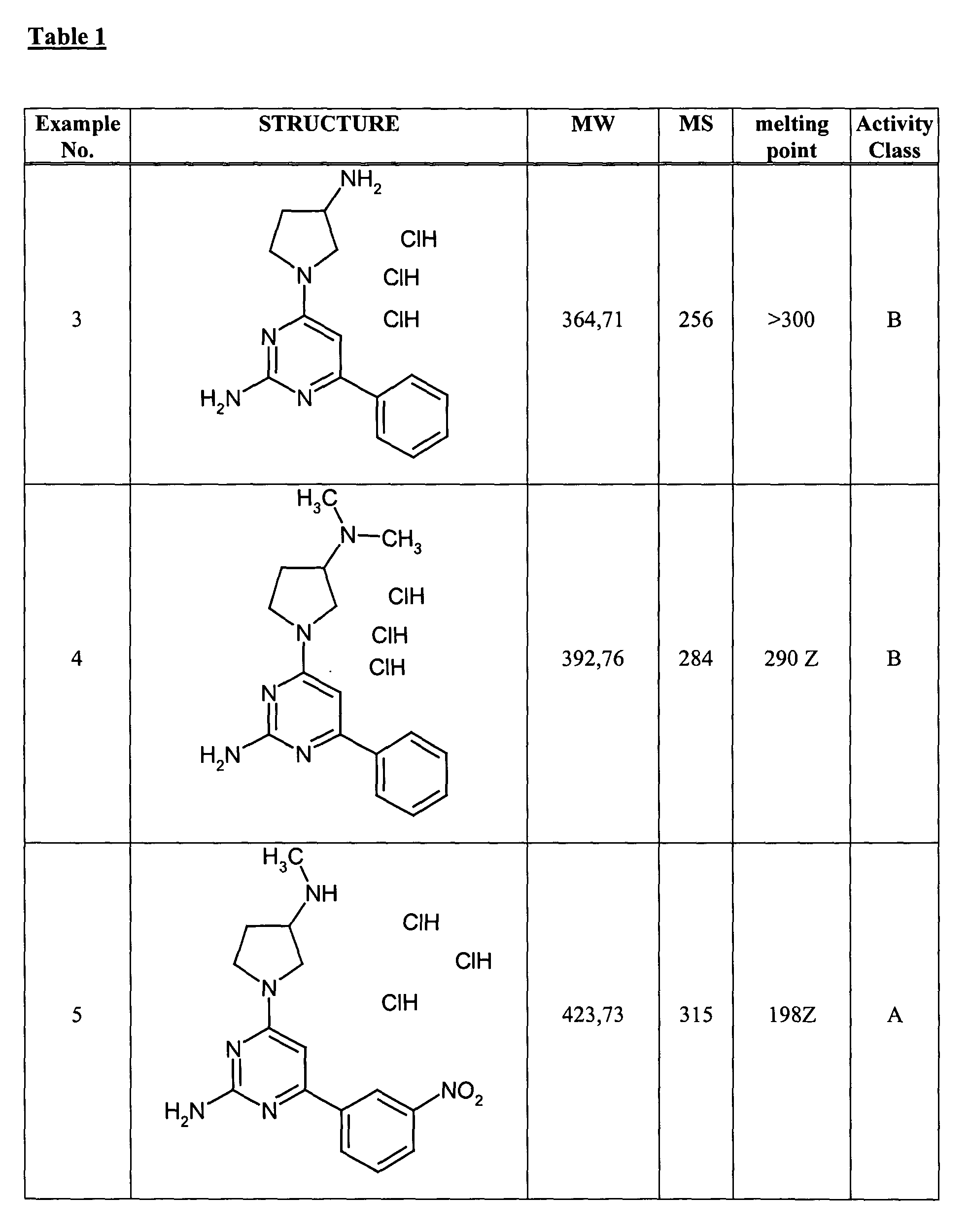

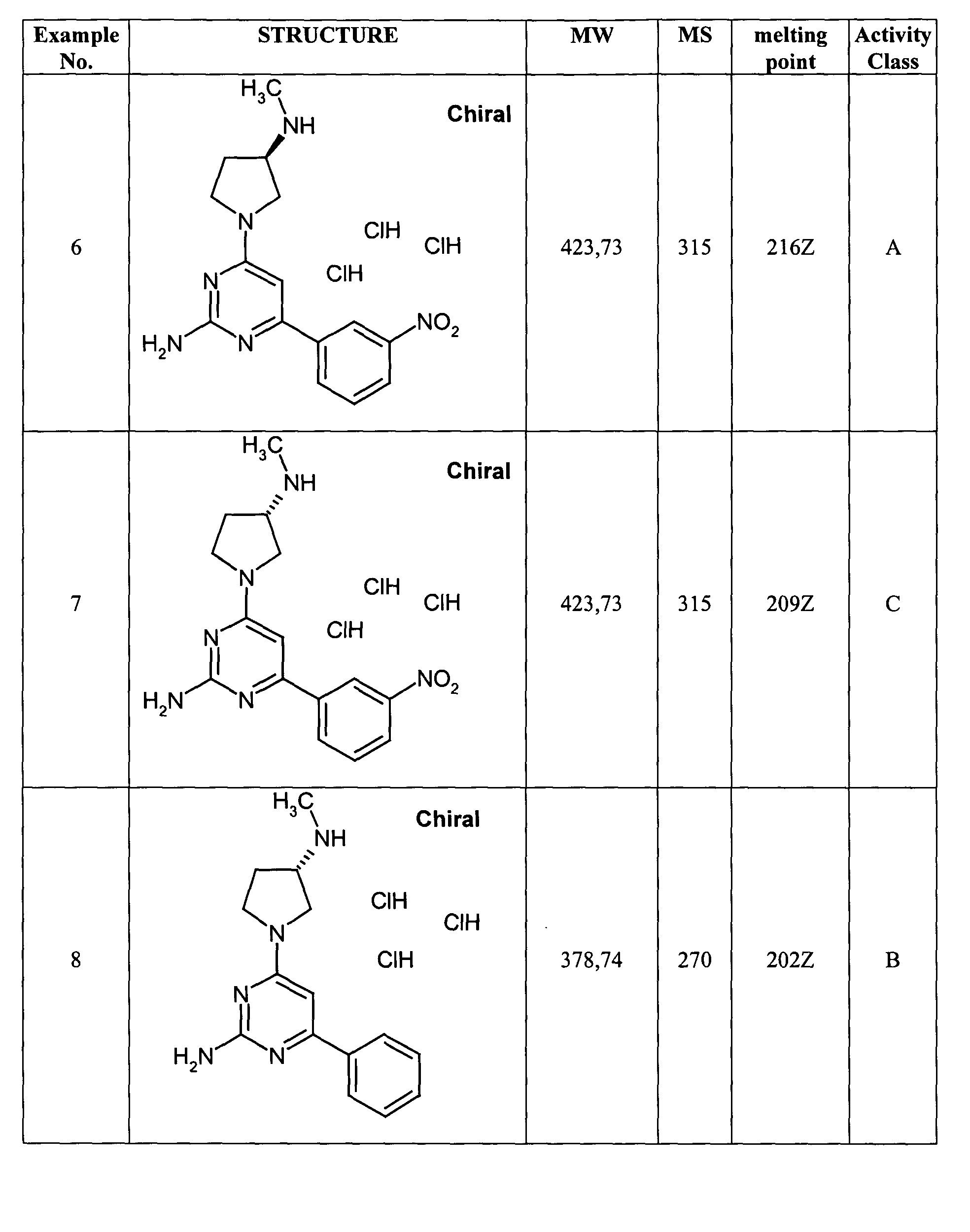

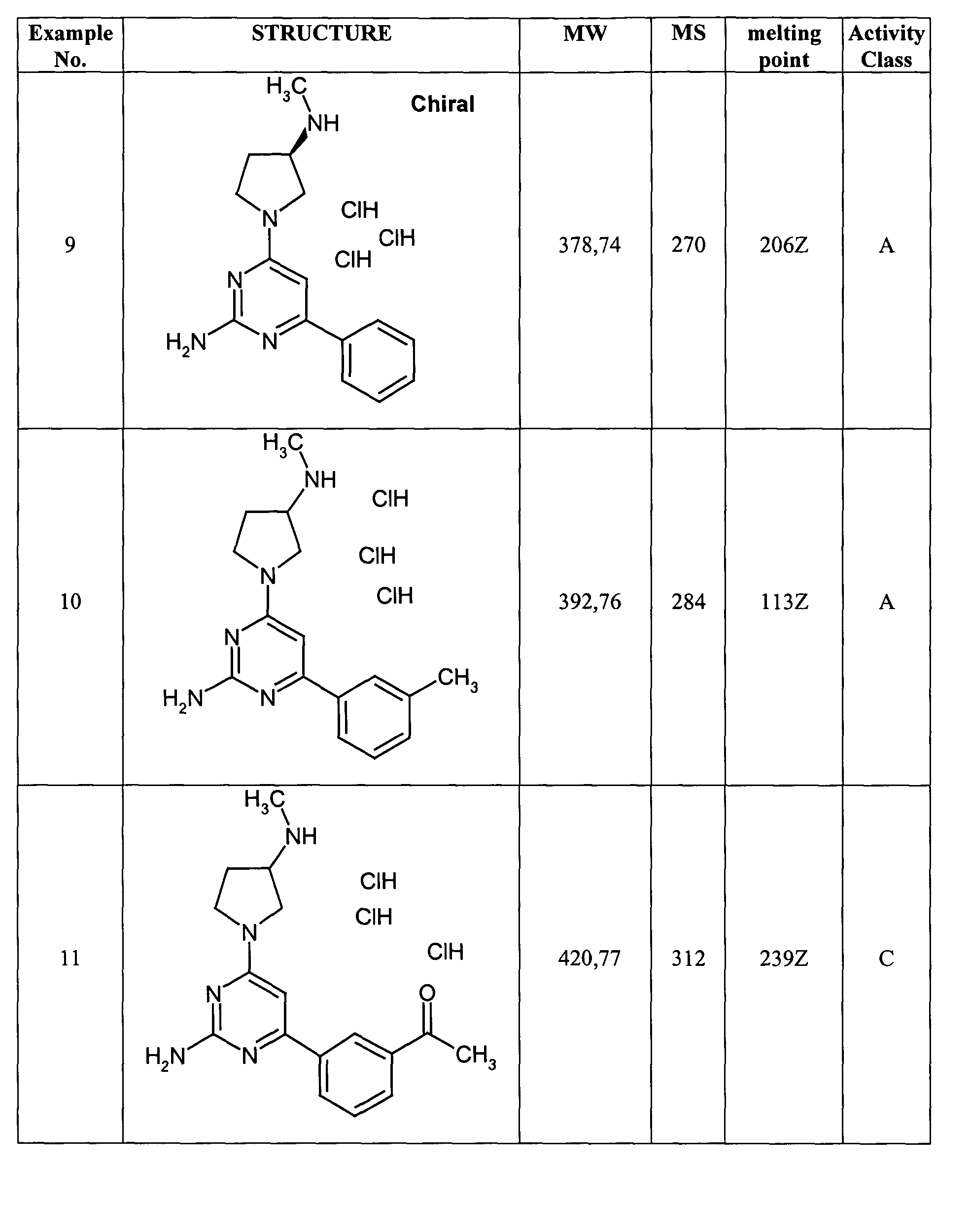

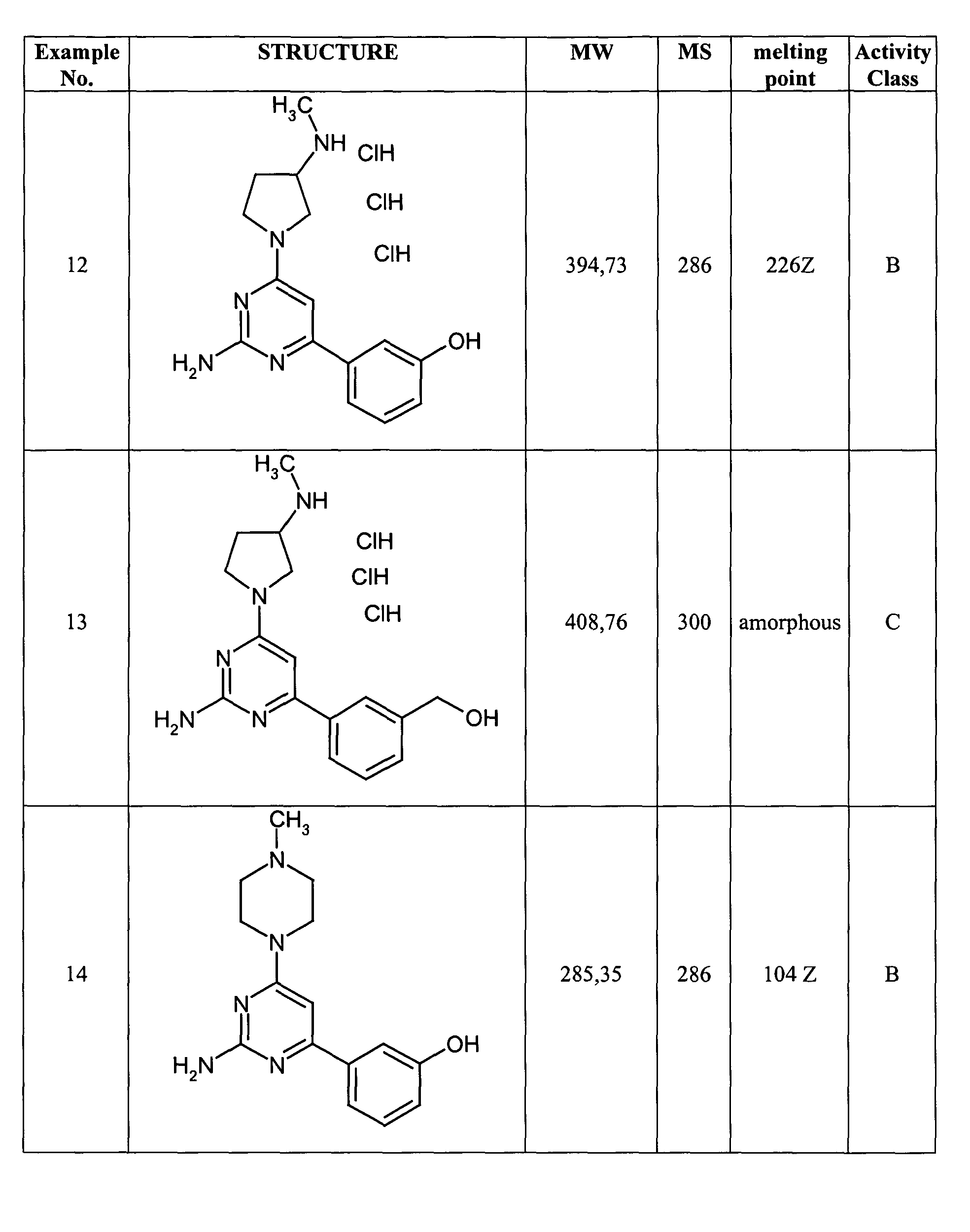

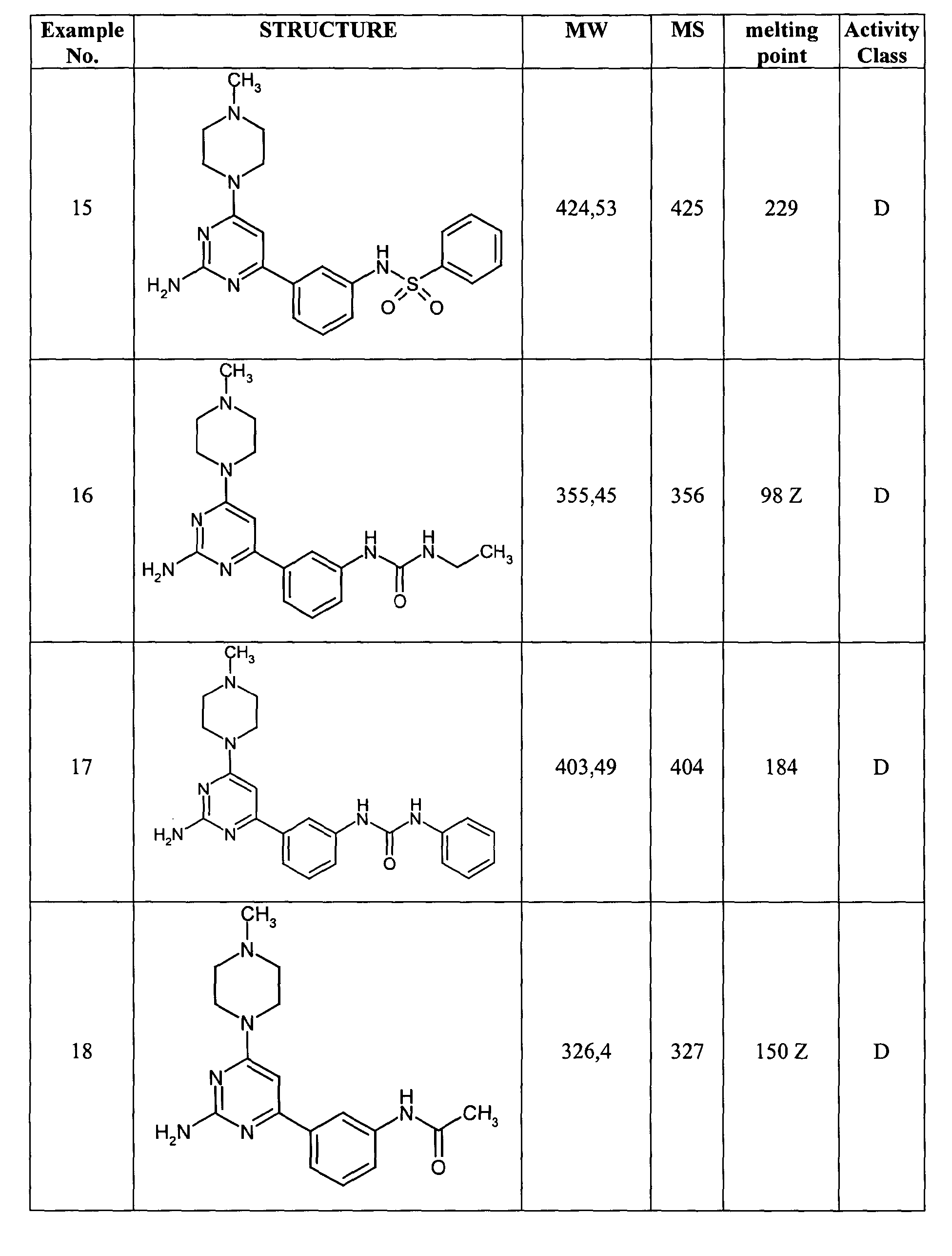

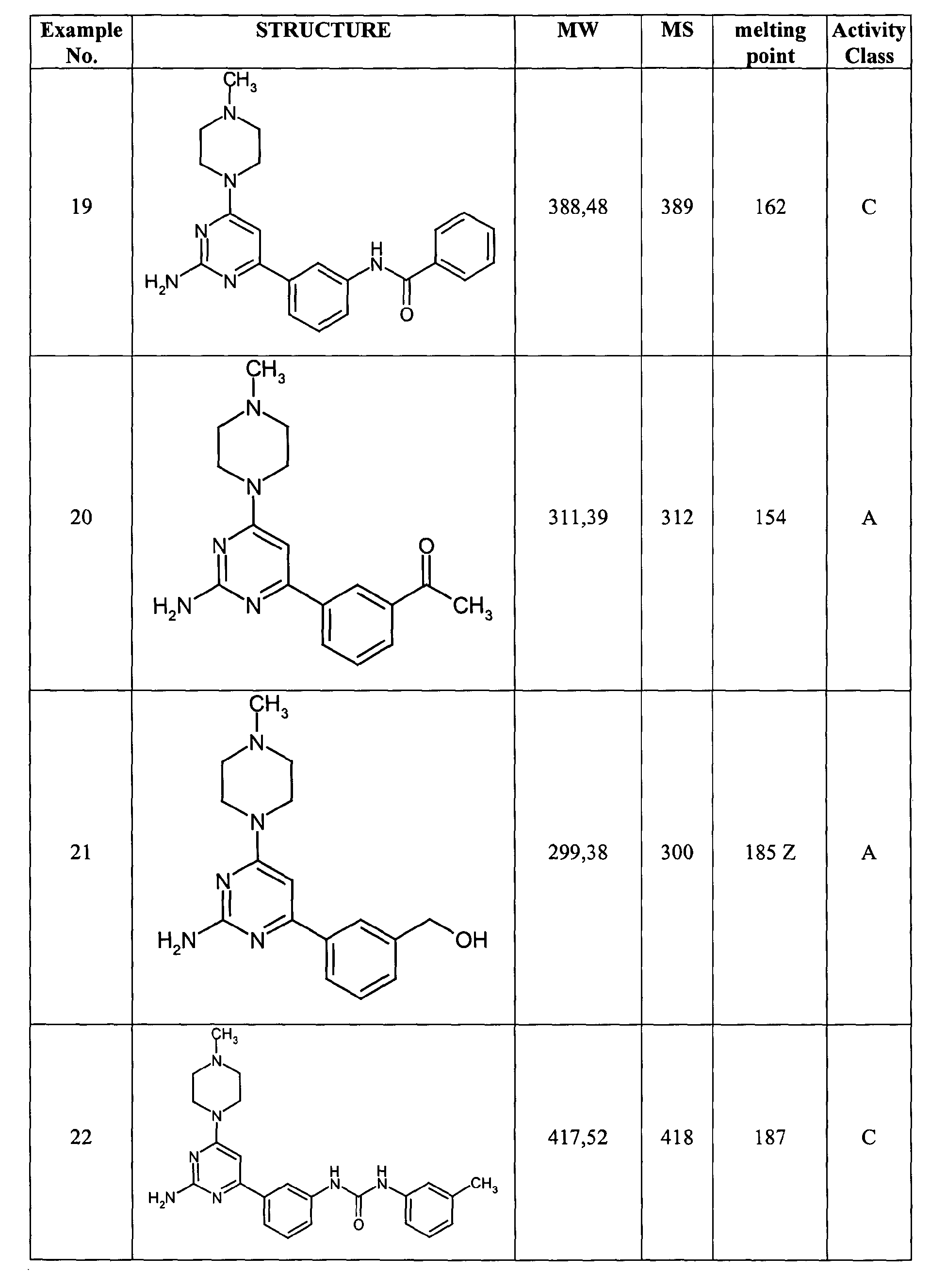

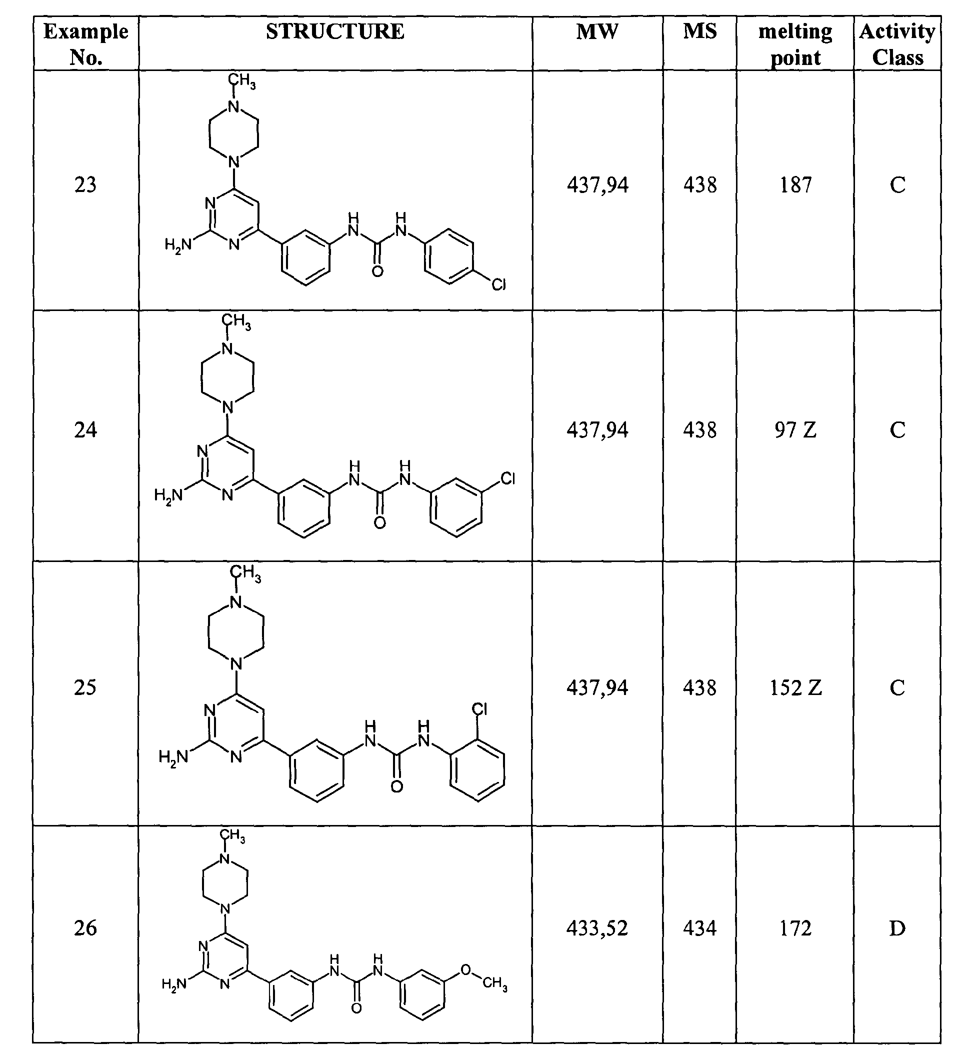

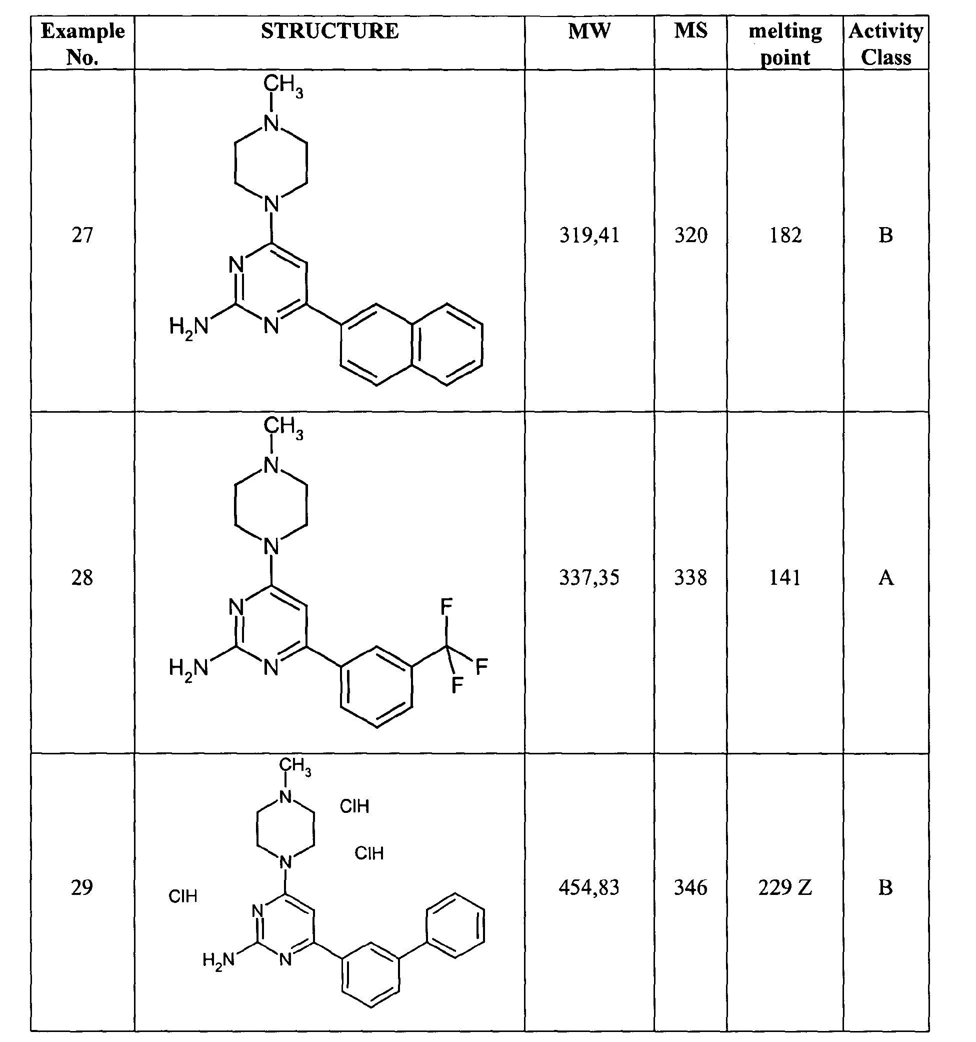

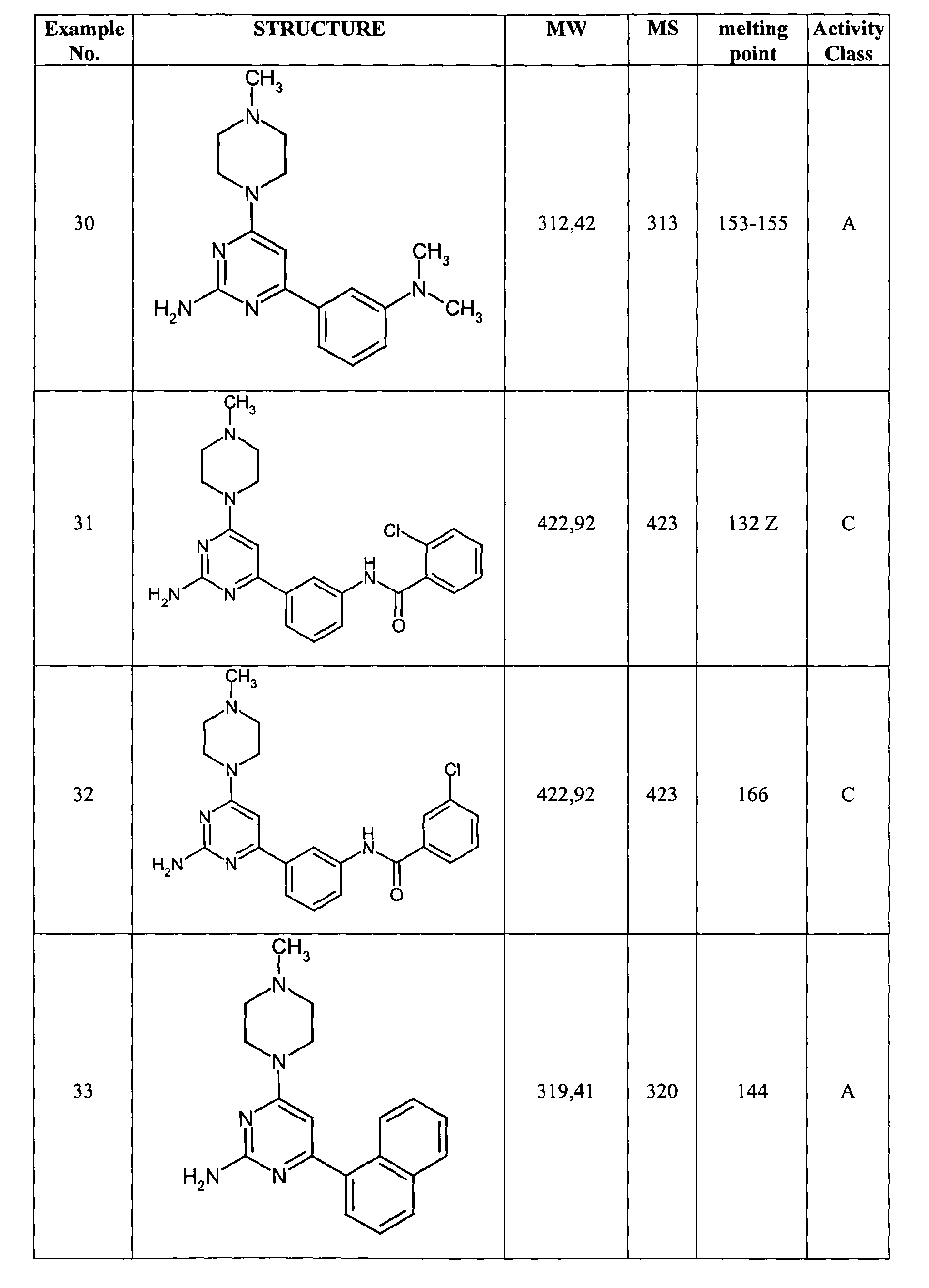

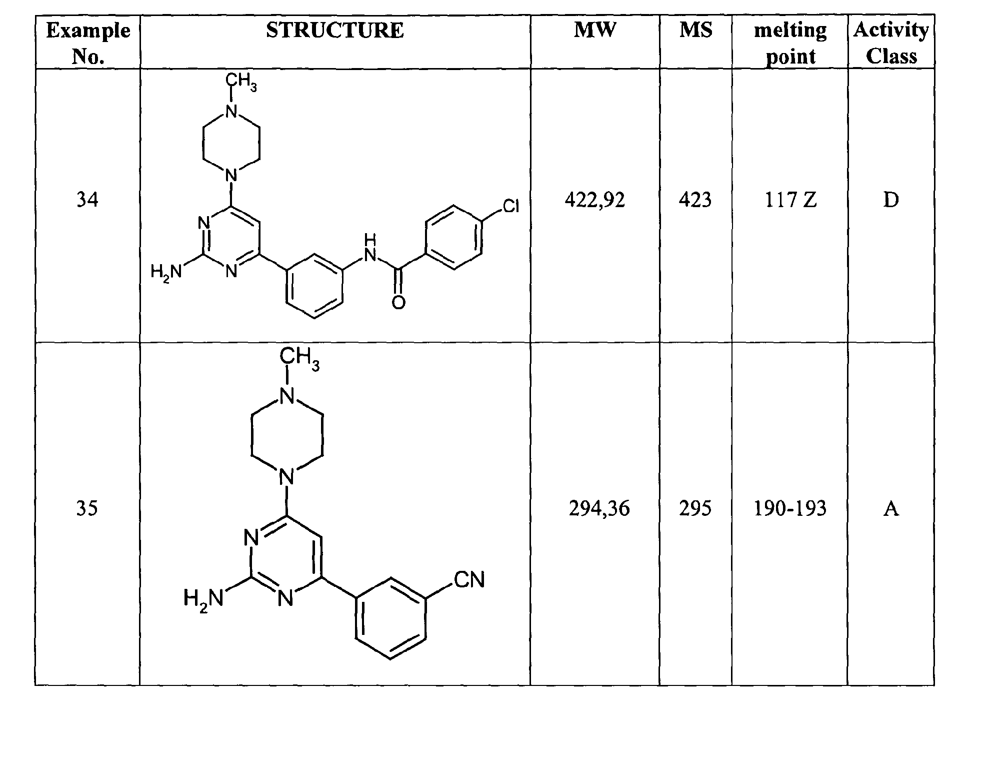

## Claims

1. A 2-aminopyrimidine derivative of the formula (I), its tautomeric or stereoisomeric form, or a salt thereof: 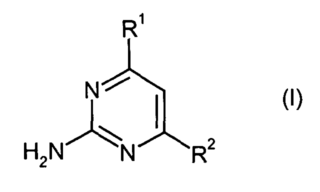 wherein
R¹ is 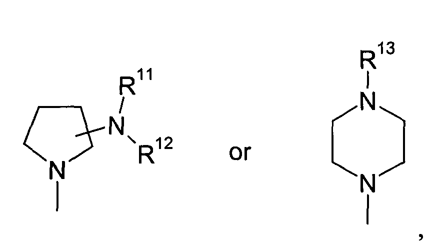 wherein
R¹¹ and R¹² independently represent hydrogen or C₁₋₆alkyl optionally substituted by halogen, cyano, hydroxy, carboxy, amino, C₁₋₆alkylamino, N,N-di(C₁₋₆alkyl)amino, C₁₋₆alkylthio, C₁₋₆-alkoxy, or C₃₋₈cycloalkyl;
R¹³ is C₁₋₆alkyl optionally substituted by halogen, cyano, hydroxy, carboxy, amino, C₁₋₆alkylamino, N,N-di(C₁₋₆alkyl)amino, C₁₋₆-alkylthio, C₁₋₆alkoxy, or C₃₋₈cycloalkyl;
R² is phenyl or naphthyl,
wherein
said phenyl and naphthyl are optionally having one or more substituents selected from the group consisting of halogen, amino, carboxy, cyano, hydroxy, nitro, phenyl, C₁₋₆alkanoyl, N-(C₁₋₆alkyl)sulfonyl-amino, N-phenylsulfonylamino, C₁₋₆alkylamino, N,N-di(C₁₋₆alkyl)-amino, N-(C₁₋₆alkanoyl)amino, C₁₋₆alkoxycarbonyl, C₁₋₆alkylthio, N-(C₁₋₆alkoxycabonyl)amino, N-arylamino, N-(aryl C₁₋₆alkyl)amino, aminocarbonyl, N-(C₁₋₆alkyl)aminocarbonyl, N,N-di(C₁₋₆alkyl)aminocarbonyl, C₃₋₈cycloalkyl, C₁₋₆alkylsulfonyl, sulfamoyl, aryl C₁₋₆-alkoxycarbonyl, C₁₋₆alkyl, C₁₋₆alkyl substituted by cyano, hydroxy, carboxy, amino, C₁₋₆alkylamino, N,N-di(C₁₋₆alkyl)amino, C₁₋₆alkylthio, C₁₋₆alkoxy, or mono-, di-, or tri- halogen, C₁₋₆alkoxy, C₁₋₆alkoxy substituted by mono-, di-, or tri- halogen, -N(R²¹)C(O)N(R²²)(R²³), and -N(R²¹)C(O) R²⁴,
with the proviso that when R¹ is 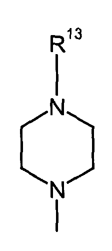 and R² is optionally substituted phenyl, said phenyl ring is substituted by at least one substituent selected from the group consisting of carboxy, cyano, hydroxy, phenyl, C₁₋₆alkanoyl, N-phenylsulfonylamino, C₁₋₆alkylamino, N,N-di(C₁₋₆alkyl)amino, N-(C₁₋₆alkanoyl)-amino, C₁₋₆alkoxycarbonyl, C₁₋₆alkylthio, N-(C₁₋₆alkoxycabonyl)-amino, N-arylamino, N-(aryl C₁₋₆alkyl)amino, aminocarbonyl, N-(C₁₋₆alkyl)aminocarbonyl, N,N-di(C₁₋₆alkyl)aminocarbonyl, C₃₋₈cycloalkyl, C₁₋₆alkylsulfonyl, aryl C₁₋₆alkoxycarbonyl, C₁₋₆alkyl substituted by cyano, hydroxy, carboxy, amino, C₁₋₆alkylamino, N,N-di(C₁₋₆-alkyl)amino, C₁₋₆alkylthio, C₁₋₆alkoxy, or mono-, di-, or tri- halogen, C₁₋₆alkoxy substituted by mono-, di-, or tri- halogen, -N(R²¹)C(O)N(R²²)(R²³), and -N(R²¹)C(O) R²⁴,
wherein
R²¹ is hydrogen or C₁₋₆alkyl;
R²² is C₁₋₆alkyl, or phenyl optionally having one or more substituents selected from the group consisting of halogen, amino, carbamoyl, cyano, hydroxy, nitro, phenyl, C₁₋₆alkanoyl, C₁₋₆alkyl optionally substituted by hydroxy or mono-, di-, or tri- halogen, and C₁₋₆alkoxy optionally substituted by mono-, di-, or trihalogen;
R²³ is hydrogen or C₁₋₆alkyl;
R²⁴ is phenyl optionally having one or more substituents selected from the group consisting of halogen, amino, carboxy, carbamoyl, cyano, hydroxy, nitro, phenyl, C₁₋₆alkanoyl, C₁₋₆alkyl optionally substituted by hydroxy or mono-, di-, or tri- halogen, and C₁₋₆alkoxy optionally substituted by mono-, di-, or tri- halogen.

2. A 2-aminopyrimidine derivative of the formula (I-i), its tautomeric or stereoisomeric form, or a salt, 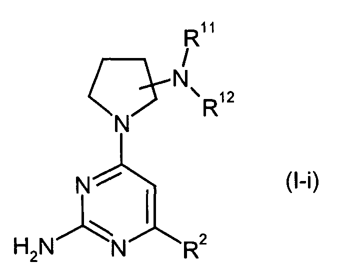 wherein
R¹¹ and R¹² independently represent hydrogen or C₁₋₆alkyl optionally substituted by halogen, cyano, hydroxy, carboxy, amino, C₁₋₆alkylamino, N,N-di(C₁₋₆alkyl)amino, C₁₋₆alkylthio, C₁₋₆alkoxy, C₃₋₈cycloalkyl;
R² is phenyl or naphthyl,
wherein
said phenyl and naphthyl are optionally having one or more substituents selected from the group consisting of halogen, amino, carboxy, cyano, hydroxy, nitro, phenyl, C₁₋₆alkanoyl, N-(C₁₋₆alkyl)-sulfonylamino, N-phenylsulfonylamino C₁₋₆alkylamino, N,N-di(C₁₋₆-alkyl)amino, N-(C₁₋₆alkanoyl)amino, C₁₋₆alkoxycarbonyl, C₁₋₆alkylthio, N-(C₁₋₆alkoxycabonyl)amino, N-arylamino, N-(arylC₁₋₆alkyl)-amino, aminocarbonyl, N-(C₁₋₆alkyl)aminocarbonyl, N,N-di(C₁₋₆-alkyl)aminocarbonyl, C₃₋₈cycloalkyl, C₁₋₆alkylsulfonyl, sulfamoyl, aryl C₁₋₆alkoxycarbonyl, C₁₋₆alkyl substituted by cyano, hydroxy, carboxy, amino, C₁₋₆alkylamino, N,N-di(C₁₋₆alkyl)amino, C₁₋₆alkylthio, C₁₋₆alkoxy, or mono-, di-, or tri- halogen, C₁₋₆alkoxy optionally substituted by mono-, di-, or tri- halogen, N(R²¹)C(O)N(R²² )(R²³) , and N(R²¹)C(O) R²⁴,
wherein
R²¹ is hydrogen or C₁₋₆alkyl;
R²² is C₁₋₆alkyl, or phenyl optionally having one or more substituents selected from the group consisting of halogen, amino, carbamoyl, cyano, hydroxy, nitro, phenyl, C₁₋₆alkanoyl, C₁₋₆alkyl optionally substituted by hydroxy or mono-, di-, or tri- halogen, and C₁₋₆alkoxy optionally substituted by mono- di-, or tri- halogen;
R²³ is hydrogen or C₁₋₆alkyl; and
R²⁴ is phenyl optionally having one or more substituents selected from the group consisting of halogen, amino, carboxy, carbamoyl, cyano, hydroxy, nitro, phenyl, C₁₋₆alkanoyl, C₁₋₆alkyl optionally substituted by hydroxy or mono-, di-, or tri- halogen, and C₁₋₆alkoxy optionally substituted by mono-, di-, or tri- halogen.

3. The 2-aminopyrimidine derivative of the formula (I-i), its tautomeric or stereoisomeric form, or a salt thereof as claimed in claim 2,
wherein
R² is phenyl or naphthyl,
wherein
said phenyl and naphthyl are optionally having one or more substituents selected from the group consisting of halogen, amino, cyano, hydroxy, nitro, phenyl, C₁₋₆alkanoyl, C₁₋₆alkylamino, N,N-di(C₁₋₆alkyl)amino, N-(C₁₋₆alkanoyl)amino, C₁₋₆alkoxycarbonyl, C₁₋₆alkylthio, C₁₋₆alkylsulfonyl, C₁₋₆alkyl, trifluoromethyl, C₁₋₆alkyl substituted by cyano, hydroxy, amino, C₁₋₆alkylamino, N,N-di(C₁₋₆alkyl)amino, or C₁₋₆-alkoxy, C₁₋₆alkoxy, N(R²¹)C(O)N(R²²)(R²³), and N(R²¹)C(O)R²⁴,
wherein
R²¹ is hydrogen or C₁₋₆alkyl;
R²² is C₁₋₆alkyl, or phenyl optionally having one or more substituents selected from the group consisting of halogen, amino, carbamoyl, cyano, hydroxy, nitro, phenyl, C₁₋₆alkanoyl, C₁₋₆alkyl optionally substituted by hydroxy or mono-, di-, or tri- halogen, and C₁₋₆alkoxy optionally substituted by mono- di-, or trihalogen;
R²³ is hydrogen or C₁₋₆alkyl; and
R²⁴ is phenyl optionally having one or more substituents selected from the group consisting of halogen, amino, carboxy, carbamoyl, cyano, hydroxy, nitro, phenyl, C₁₋₆alkanoyl, C₁₋₆alkyl optionally substituted by hydroxy or mono-, di-, or tri- halogen, and C₁₋₆alkoxy optionally substituted by mono-, di-, or tri- halogen.

4. The 2-aminopyrimidine derivative of the formula (I-i), its tautomeric or stereoisomeric form, or a salt thereof as claimed in claim 2,
wherein
R¹¹ and R¹² independently represent hydrogen or methyl.

5. The 2-aminopyrimidine derivative of the formula (I-i), its tautomeric or stereoisomeric form, or a salt thereof as claimed in claim 2, wherein said derivative is selected from the group consisting of the following compounds:
4-(3-aminopyrrolidin-1-yl)-6-phenylpyrimidin-2-amine trihydrochloride;
4-[3-(dimethylamino)pyrrolidin-1-yl]-6-phenylpyrimidin-2-amine trihydrochloride;
4-[3-(methylamino)pyrrolidin-1-yl]-6-phenylpyrimidin-2-amine trihydrochloride;
4-[3-(methylamino)pyrrolidin-1-yl]-6-(3-nitrophenyl)pyrimidin-2-amine trihydrochloride;
4-[(3R)-3-(methylamino)pyrrolidin-1-yl]-6-(3-nitrophenyl)pyrimidin-2-amine trihydrochloride;
4-[(3S)-3-(methylamino)pyrrolidin-1-yl]-6-(3-nitrophenyl)pyrimidin-2-amine trihydrochloride;
4-[(3S)-3-(methylamino)pyrrolidin-1-yl]-6-phenylpyrimidin-2-amine trihydrochloride;
4-[(3R)-3-(methylamino)pyrrolidin-1-yl]-6-phenylpyrimidin-2-amine trihydrochloride;
4-[3-(methylamino)pyrrolidin-1-yl]-6-(3-methylphenyl)pyrimidin-2-amine trihydrochloride;
1-(3-{2-amino-6-[3-(methylamino)pyrrolidin-1-yl]pyrimidin-4-yl}phenyl)ethanone trihydrochloride;
3-{2-amino-6-[3-(rnethylamino)pyrrolidin-1-yl]pyrimidin-4-yl}phenol trihydrochloride;
(3- {2-amino-6-[3-(methylamino)pyrrolidin-1-yl]pyrimidin-4-yl}phenyl)methanol trihydrochloride; and
3- {2-amino-6-[3-(methylamino)pyrrolidin-1-yl]pyrimidin-4-yl}benzonitrile trihydrochloride.

6. A 2-aminopyrimidine derivative of the formula (I-ii), its tautomeric or stereoisomeric form, or a salt, 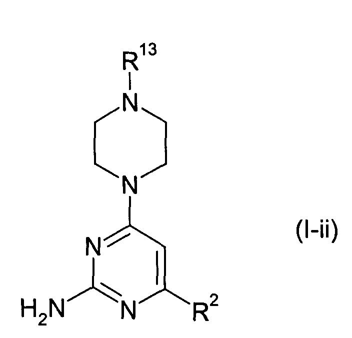 wherein
R¹³ is C₁₋₆alkyl optionally substituted by halogen, cyano, hydroxy, carboxy, amino, C₁₋₆alkylamino, N,N-di(C₁₋₆alkyl)amino, C₁₋₆alkylthio, C₁₋₆alkoxy, C₃₋₈cycloalkyl;
R² is phenyl having one or more substituents selected from the group consisting of carboxy, cyano, hydroxy, phenyl, C₁₋₆alkanoyl, N-(C₁₋₆alkyl)sulfonylamino, N-phenylsulfonylamino, C₁₋₆alkylamino, N,N-di(C₁₋₆alkyl)amino, N-(C₁₋₆alkanoyl)amino, C₁₋₆alkoxycarbonyl, C₁₋₆alkylthio, N-(C₁₋₆alkoxycabonyl)amino, N-arylamino, N-(aryl C₁₋₆alkyl)amino, aminocarbonyl, N-(C₁₋₆alkyl)aminocarbonyl, N,N-di(C₁₋₆alkyl)aminocarbonyl, C₃₋₈cycloalkyl, C₁₋₆alkylsulfonyl, aryl C₁₋₆alkoxycarbonyl, C₁₋₆alkyl substituted by cyano, hydroxy, carboxy, amino, C₁₋₆alkylamino, N,N-di(C₁₋₆alkyl)amino, C₁₋₆alkylthio, C₁₋₆alkoxy, or mono-, di-, or tri- halogen, C₁₋₆alkoxy substituted by mono-, di-, or tri- halogen, -N(R²¹)C(O)N(R²²)(R²³), and N(R²¹)C(O) R²⁴, or naphthyl optionally having one or more substituents selected from the group consisting of halogen, amino, carboxy, cyano, hydroxy, nitro, phenyl, C₁₋₆alkanoyl, N-phenylsulfonylamino, C₁₋₆alkylamino, N,N-di(C₁₋₆alkyl)amino, N-(C₁₋₆alkanoyl)amino, C₁₋₆alkoxycarbonyl, C₁₋₆alkylthio, N-(C₁₋₆alkoxycabonyl)amino, N-arylamino, N-(aryl C₁₋₆alkyl)amino, aminocarbonyl, N-(C₁₋₆alkyl)-aminocarbonyl, N,N-di(C₁₋₆alkyl)aminocarbonyl, C₃₋₈cycloalkyl, C₁₋₆alkylsulfonyl, sulfamoyl, aryl C₁₋₆alkoxycarbonyl, C₁₋₆alkyl optionally substituted by cyano, hydroxy, carboxy, amino, C₁₋₆alkylamino, N,N-di(C₁₋₆alkyl)amino, C₁₋₆alkylthio, C₁₋₆alkoxy, or mono-, di-, or tri- halogen, C₁₋₆alkoxy optionally substituted by amono-, di-, or tri- halogen, -N(R²¹)C(O)N(R²²)(R²³), and N(R²¹)C(O) R²⁴ ,
wherein
R²¹ is hydrogen or C₁₋₆alkyl;
R²² is C₁₋₆alkyl, or phenyl optionally having one or more substituents selected from the group consisting of halogen, amino, carbamoyl, cyano, hydroxy, nitro, phenyl, C₁₋₆alkanoyl, C₁₋₆alkyl optionally substituted by hydroxy or mono-, di-, or tri- halogen, and C₁₋₆alkoxy optionally substituted by mono-, di-, or tri- halogen;
R²³ is hydrogen or C₁₋₆alkyl;
R²⁴ is phenyl optionally having one or more substituents selected from the group consisting of halogen, amino, carboxy, carbamoyl, cyano, hydroxy, nitro, phenyl, C₁₋₆alkanoyl, C₁₋₆alkyl optionally substituted by hydroxy or mono-, di-, or tri- halogen, and C₁₋₆alkoxy optionally substituted by amono-, di-, or tri- halogen.

7. The 2-aminopyrimidine derivative of the formula (I-ii), its tautomeric or stereoisomeric form, or a salt thereof as claimed in claim 5, wherein
R² is phenyl having one or more substituents selected from the group consisting of cyano, hydroxy, phenyl, C₁₋₆alkanoyl, C₁₋₆alkylamino, N,N-di(C₁₋₆alkyl)amino, N-(C₁₋₆alkanoyl)amino, C₁₋₆alkoxycarbonyl, C₁₋₆alkylthio, C₁₋₆alkylsulfonyl, trifluoromethyl, C₁₋₆alkyl substituted by cyano, hydroxy, amino, C₁₋₆alkylamino, N,N-di(C₁₋₆alkyl)amino, or C₁₋₆alkoxy, and C₁₋₆alkoxy,or naphthyl optionally having one or more substituents selected from the group consisting of halogen, amino, cyano, hydroxy, nitro, phenyl, C₁₋₆alkanoyl, C₁₋₆alkylamino, N,N-di(C₁₋₆alkyl)amino, N-(C₁₋₆alkanoyl)amino, C₁₋₆alkoxycarbonyl, C₁₋₆alkylthio, C₁₋₆alkylsulfonyl, C₁₋₆alkyl, trifluoromethyl, C₁₋₆alkyl substituted by cyano, hydroxy, amino, C₁₋₆alkylamino, N,N-di(C₁₋₆alkyl)amino, or C₁₋₆alkoxy, and C₁₋₆alkoxy.

8. The 2-aminopyrimidine derivative of the formula (I-ii), its tautomeric or stereoisomeric form, or a salt thereof as claimed in claim 5, wherein said derivative is selected from the group consisting of the following compounds:
3-[2-amino-6-(4-methylpiperazin-1-yl)pyrimidin-4-yl]phenol;
1-{3 -[2-amino-6-(4-methylpiperazin-1 -yl)pyrimidin-4-yl]phenyl} ethanone;
{3-[2-amino-6-(4-methylpiperazin-1-yl)pyrimidin-4-yl]phenyl} methanol;
4-(4-methylpiperazin-1-yl)-6-[3-(trifluoromethyl)phenyl]pyrimidin-2-amine;
4-biphenyl-3-yl-6-(4-methylpiperazin-1-yl)pyrimidin-2-amine trihydrochloride;
4-[3-(dimethylamino)phenyl]-6-(4-methylpiperazin-1-yl)pyrimidin-2-amine;
4-(4-methylpiperazin-1-yl)-6-(1-naphthyl)pyrimidin-2-amine; and
3-[2-amino-6-(4-methylpiperazin-1-yl)pyrimidin-4-yl]benzonitrile.

9. A medicament comprising the 2-aminopyrimidine derivative, its tautomeric or stereoisomeric form, or a physiologically acceptable salt thereof as claimed in any one of claim 1 to 8 as an active ingredient.

10. The medicament as claimed in claim 9, further comprising one or more pharmaceutically acceptable excipients.

11. The medicament as claimed in claim 9, wherein said 2-aminopyrimidine derivative, its tautomeric or stereoisomeric form, or a physiologically acceptable salt thereof is a histamine H4 receptor antagonist.

12. The medicament as claimed in claim 9 for the treatment and/or prevention of an inflammatory disorder or disease.

13. The medicament as claimed in claim 12, wherein said inflammatory disorder or disease is asthma, rhinitis, allergic diseases or chronic obstructed pulmonary disease (CORD).

14. The medicament as claimed in claim 9 for the treatment and/or prevention of an immunological disorder or disease.

15. The medicament as claimed in claim 14, wherein said immunological disorder or disease is rheumatoid arthritis or atherosclerosis.

16. Use of a compound according to any one of claim 1 to 8 for manufacturing a medicament for the treatment and/or prevention of an inflammatory disorder or disease.

17. Use of a compound according to any one of claim 1 to 8 for manufacturing a medicament for the treatment and/or prevention of an immunological disorder or disease.

18. Process for controlling an inflammatory disorder or disease in humans and animals by administration of a histamine H4 receptor antagonisticly effective amount of a compound according to any one of claim 1 to 8.

19. Process for controlling an immunological disorder or disease in humans and animals by administration of a histamine H4 receptor antagonisticly effective amount of a compound according to any one of claim 1 to 8.
